# EUROPÄISCHE PATENTSCHRIFT

(11) **EP 0 950 065 B1**
(45) Veröffentlichungstag und Bekanntmachung des Hinweises auf die Patenterteilung: **19.02.2003**
(21) Anmeldenummer: 97942949.5
(22) Anmeldetag: 18.09.1997
(51) Int. Cl.: C07K 14/47, C07K 14/705

(54) **PEPTIDE ALS DIAGNOSTIKUM UND THERAPEUTIKUM FÜR AUTOIMMUNERKRANKUNGEN**
PEPTIDE AS DIAGNOSTIC AND THERAPEUTIC AGENT FOR AUTOIMMUNE DISEASES
PEPTIDES SERVANT D'AGENT DIAGNOSTIQUE ET D'AGENT THERAPEUTIQUE POUR LES MALADIES AUTO-IMMUNES

(30) Priorität: 18.09.1996 DE 19638108
(43) Veröffentlichungstag der Anmeldung: 20.10.1999
(73) Patentinhaber: Wildner, Gerhild, 80636 München (DE)
(72) Erfinder: Wildner, Gerhild, 80636 München (DE)
(74) Vertreter: Wibbelmann, Jobst, Dr., Dipl.-Chem.
(86) Internationale Anmeldenummer: EP9705124
(87) Internationale Veröffentlichungsnummer: WO98012221

(56) Entgegenhaltungen:
- WO-A-93/17699
- WO-A-94/05303
- G WILDNER & S R THURAU: "Cross-reactivity between an HLA-B27-derived peptide and a retinal autoantigen peptide; a clue to major histocompatibility complex association with autoimmune disease" EUR. J. IMMUNOL., Bd. 24, Nr. 11, November 1994, WEINHEIM, DE, Seiten 2579-2585, XP002054583 in der Anmeldung erwähnt
- CHEMICAL ABSTRACTS, vol. 124, no. 13, 25.März 1996 Columbus, Ohio, US; abstract no. 172883, XP002054584 & E NOESSNER ET AL.: "HLA-derived peptides which inhibit T cell function bind to members of the heat-shock protein 70 family" J. EXP. MED., Bd. 183, Nr. 2, 1996, Seiten 339-348,
- CHEMICAL ABSTRACTS, vol. 113, no. 19, 5.November 1990 Columbus, Ohio, US; abstract no. 169984, B P CHEN ET AL.: "Cytotoxic T cell recognition of an endogenous class I HLA peptide presented by a class II HLA molecule" XP002054585 & J. EXP. MED., Bd. 172, Nr. 3, 1990, Seiten 779-799,
- CHEMICAL ABSTRACTS, vol. 122, no. 15, 10.April 1995 Columbus, Ohio, US; abstract no. 184950, R M CHICZ ET AL.: "Self-peptides bound to the type I diabetes associated class II MHC molecules HLA-DQ1 and HLA-DQ8" XP002054586 & INT. IMMUNOL., Bd. 6, Nr. 11, 1994, Seiten 1639-1649,

## Beschreibung

Die vorliegende Erfindung betrifft von den α1- oder α2-Domänen von HLA-B27 bzw. humanem Keratin VI abgeleitete Peptide sowie die Verwendung bestimmter, von den α1- oder α2-Domänen von HLA-B27 bzw. humanem Keratin VI abgeleiteter Peptide zur Diagnose und Behandlung von HLA-abhängigen Autoimmunerkrankungen.

Autoimmunerkrankungen, wie beispielsweise Rheuma, Psoriasis (Schuppenflechte), multiple Sklerose, Colitis ulcerosa, Autoimmun-Uveitis, sind Erkrankungen, bei denen das körpereigene Abwehrsystem (Immunsystem) Strukturen des eigenen Organismus nicht mehr toleriert, sondern wie einen eingedrungenen Erreger attackiert. Dies hat zur Folge, daß körpereigenes Gewebe geschädigt oder sogar zerstört wird und dadurch Schmerzen und körperliche Ausfallserscheinungen auftreten: beispielsweise steife Gelenke bei Rheuma; entzündliche, schuppige und z.T. großflächige Hautläsionen bei Psoriasis; allgemeine Bewegungsschwäche, Inkontinenz, Sprachstörungen bei multipler Sklerose; schwere Durchfälle, Ernährungsstörungen bis hin zur Darmperforation mit tödlichem Ausgang bei Colitis ulcerosa; bei Uveitis Verminderung der Sehstärke, die bis zur Erblindung führen kann.

Autoimmunerkrankungen verlaufen in fast allen Fällen chronisch, entweder schubweise wiederkehrend oder permanent schleichend. Die durch das Immunsystem zerstörten Gewebe können in den seltensten Fällen regeneriert werden. Bei rheumatischen Erkrankungen kann die autoaggressive Immunantwort zu sehr schmerzhaften Gelenksentzündungen, zu Degenerationen der Gelenke bis zur Invalidität führen. Die teilweise großflächigen, stark juckenden und auffälligen Hautläsionen bei Psoriasis führen bei den Patienten sehr häufig zu psychischen Problemen, v.a. im zwischenmenschlichen Bereich.

Die Ursachen für die Entstehung solcher Autoimmunerkrankungen sind noch weitgehend unbekannt. In manchen Fällen vermutet man als an der Auslösung der Autoimmunerkrankung maßgebend beteiligtes Ereignis Infektionen durch Bakterien oder Viren, durch die das Immunsystem fehlgeleitet wird. Sowohl bei rheumatischen Erkrankungen als auch bei Psoriasis konnte gezeigt werden, daß die zelluläre Immunantwort eine wichtige Rolle bei der Pathogenese spielt, aber auch Antikörperantworten konnten in beiden Fällen nachgewiesen werden.

Die konventionellen Therapiemaßnahmen, die im Falle von Autoimmunerkrankungen eingeleitet werden, betreffen nicht nur den selbstreaktiven ("autoaggressiven") Teil des Immunsystems, sondern supprimieren generell immunreaktive Zellen, die eigentlich zur Abwehr von eingedrungenen Erregern oder von Tumorzellen dienen sollten. Neben einer verringerten allgemeinen Immunabwehr und einer möglichen Entstehung von Tumoren haben die konventionellen Immunsuppressiva wie Corticosteroide, Cyclosporin A und Zytostatika auch viele nicht-immunologische und z.T. lebensbedrohliche Nebenwirkungen zur Folge.

Für viele (Autoimmun-) Erkrankungen wurde eine erbliche Disposition aufgrund statistischer Korrelation der Krankheit mit bestimmten Antigenen des Haupthistokompatibilitätskomplexes (MHC) oder, synonym, HLA (humane Leukozytenantigene, ursprünglich als Transplantationsantigene entdeckt) vermutet. Der Mechanismus wurde als eine Verwechslung von Erreger-Antigenen (Bakterien, Viren) mit körpereigenen HLA-Antigenen durch das Immunsystem postuliert.

Das HLA-B27-Antigen stellt offenbar eine Besonderheit dar, da es bei bestimmten Autoimmunerkrankungen besonders gehäuft auftritt, wie z.B. bei Iritis, Psoriasis sowie rheumatischen Erkrankungen, wie Morbus Bechterew (ankylosierende Spondylitis), Psoriasis-Arthritis (Arthritis psoriatica) und juveniler rheumatoider Arthritis bei Knaben.

Bei der Autoimmunuveitis konnte bereits gezeigt werden (Wildner, G. & Thurau, R., Eur. J. Immunol. 24, 2579-2585 (1994)), daß das HLA-Klasse I-Antigen seine Rolle bei der Pathogenese nicht als antigenpräsentierendes Element spielt, sondern in Form eines Peptids selbst als Antigen, präsentiert auf HLA-Klasse II oder anderen Molekülen, fungiert. Über Kreuzreaktivitäten mit bestimmten Autoantigenen (z.B. retinales S-Antigen bei der Uveitis) läßt sich dann auch die Organspezifität der autoaggressiven Immunreaktion erklären.

Autoimmunerkrankungen, bei denen HLA-Antigene eine wichtige Rolle bei der Pathogenese der Erkrankung spielen, werden im Zusammenhang mit der vorliegenden Erfindung als "HLA-abhängige" Autoimmunerkrankungen bezeichnet.

Bei HLA-abhängigen Erkrankungen des rheumatischen Formenkreises (ankylosierende Spondylitis, reaktive Arthritis, Psoriasis-Arthritis) waren die hauptverdächtigen Antigene bisher bakterielle Proteine, die auf HLA-Klasse I und -II, in einigen Fällen präferentiell auf HLA-B27 präsentiert werden sollten, oder auch (gelenkspezifisches) Collagen Typ II, bei dem aber der Zusammenhang mit HLA-B27 nicht hergestellt werden konnte. Bei Psoriasis werden Streptococcus-Proteine und möglicherweise Keratinozytenproteine als Antigene vermutet.

Zelluläre Immunreaktionen von Rheuma- und Psoriasispatienten auf Peptide aus der Sequenz von HLA-B27 bzw. Hautkeratin legen nahe, daß es sich bei diesen Peptiden um potentielle Autoantigenpeptide handelt, also um Peptide, die nicht mehr als körpereigen oder "selbst" erkannt werden. Zelluläre Immunreaktionen auf die ersten beiden Domänen von HLA-B27 konnten bereits 1986 beschrieben werden (Wildner, G. et al., Abstract 2.33.13, Sixth International Congress of Immunology, Toronto, Kanada, 6. bis 11. Juli 1986; Wildner, G., Dissertation, LMU München, 1987; Wildner, G., et al., Mol. Immunol. 26, 33 - 40, 1989).

In den letzten Jahren wurden viele Versuche unternommen, regulierend in das Immunsystem einzugreifen, um das "normale" Gleichgewicht zwischen Selbsttoleranz und Abwehr von Krankheitserregern wieder herzustellen. Dabei sollen nicht alle Immunreaktionen unspezifisch unterdrückt werden, sondern nur der Zweig der Immunantwort, der autoaggressiv wirksam ist, getroffen werden. Man hofft daher, Nebenwirkungen wie die Erhöhung des Infektionsrisikos oder Tumorbildung zu vermeiden.

In diesem Zusammenhang ist ein Vergleich der physiologischen Mechanismen, die zu Autoimmunerkrankungen oder zu akuter oder chronischer Transplantatabstoßung führen, soweit sie bereits bekannt sind, interessant. Diese Mechanismen werden gleichermaßen bereits seit einiger Zeit mit dem Ziel, in diesen Fällen Abhilfe zu schaffen, intensiv untersucht. So wurde festgestellt, daß bei der akuten Transplantatabstoßung, cytotoxische Aktivität insbesondere durch CD8-positive cytotoxische T-Lymphozyten als primäres Ereignis im Vordergrund steht, wohingegen bei der chronischen Transplantatabstoßung die cytotoxische Aktivität nur von sekundärer Bedeutung zu sein scheint. Auf Ebene der zellulären Immunantwort scheinen hier z.B. CD4-positive T-Lymphozyten, insbesondere Helfer-T-Lymphozyten, Suppressor-T-Lymphozyten, Zytokine,.. von größerer Bedeutung zu sein. Ähnliches wie bei der chronischen Transplantatabstoßung gilt nach derzeitigem Wissenstand für Autoimmunerkrankungen.

Mittel zur Behandlung von Autoimmunerkrankungen werden dementsprechend im Gegensatz zu Mitteln zur Verhütung der akuten Transplantatabstoßung nicht primär auf die Modulation cytotoxischer T-Lymphozytenaktivität abzielen, sondern ein breiteres Wirkungsspektrum auf Ebene der zellulären wie der humoralen Immunantwort anstreben.

Eine Lösung der vorstehend definierten Aufgabe in Bezug auf HLA-abhängige Autoimmunerkrankungen, inbesondere HLA-Klasse I-, aber auch HLA-Klasse II-assoziierte Autoimmunerkrankungen sowie insbesondere jene, die speziell mit HLA-B27 assoziiert sind, besteht in der erfindungsgemäßen Verwendung bestimmter, von den α1- oder α2-Domänen von HLA-B27 bzw. humanem Keratin VI abgeleiteter Peptide zur Diagnose und Behandlung von HLA-abhängigen Autoimmunerkrankungen.

Bereits in 1986, 1987 und 1989 wurden von Wildner, G., et al. (a.a.O.) Fusionsproteine beschrieben, die von den α1- oder α2-Domänen von HLA-B27 abgeleitete Peptide umfaßten. Auch in WO 88/05784 von Krensky et al., WO 93/17699 von Clayberger, C.A., und Krensky, A.M., WO 95/26979 von Clayberger, C.A., et al., sowie in den Veröffentlichungen von Clayberger, C.A., und Krensky, A.M., in Current Opinion in Immunology 7, 644-648, (1995) und von Nößner, E. et al. in J. Exp. Med. 183, 339-348, (1996) werden von den α1- oder α2-Domänen von HLA-Klasse I-Molekülen abgeleitete Peptide beschrieben.

Entsprechend den Dokumenten WO 88/05784, WO 93/17699, WO 95/26979, Current Opinion in Immunology (1995, a.a.O.) und J. Exp. Med. (1996, a.a.O.) sind jeweils bestimmte Gruppen der offenbarten Peptide gedacht für
- die Modulation der zytotoxischen/zytolytischen Aktivität von zytotoxischen T-Zellen, insbesondere zytotoxischer T-Lymphozyten (CTL), in Patienten, und zwar in Form
   -- einer Inhibition von CTL-Aktivität,
      - durch Bindung der Peptide an CTL, insbesondere über T-Zell-Rezeptoren, die zu einer Inhibition der Lyse von Zielzellen durch CTL führt (wahrscheinlich über Kompetition mit dem normalen Liganden für die Bindung an den CTL (Current Opinion Immunol. (1995), a.a.O.) und/oder
      - über Bindung an Mitglieder der Heat-Shock-Protein-Familie HSP 70 in einer mutmaßlich der Wirkung anderer immunmodulatorischer Verbindungen, FK506 und Cyclosporin A, ähnlichen Weise, da die beteiligten Heat-Shock-Proteine bislang auf der Oberfläche der betroffenen T-Zellen nicht nachgewiesen werden konnten (J. Exp. Med (1996), a.a.O.),
      - durch Inhibition/Blockierung der CTL-Differenzierung (WO 93/17699), oder
   -- einer Sensibilisierung von MHC-restringierten Zielzellen gegenüber CTL/Stimulierung der CTL-Aktivität, wahrscheinlich über Präsentation der Peptide im MHC-Kontext gegenüber den CTL,
- die Identifizierung von CTL bzw. die Diagnose des Vorliegens bestimmter CTL über die Bindung an die entsprechenden Peptide,
- die Entfernung bestimmter CTL-Populationen aus einer T-Zell-Zusammensetzung über die Bindung an die entsprechenden Peptide.

Der Anwendungsbereich der beschriebenen Peptide wird in sämtlichen Dokumenten im Bereich der Transplantationsmedizin, und zwar in Verbindung mit akuter Transplantatabstoßung (siehe auch WO 95/26979 von Clayberger et al. sowie Clayberger, C.A. und Krensky, A.M., Current Opinion in Immunology 7, 644-648, (1995) und Nößner, E. et al., J. Exp. Med. 183, 339-348, (1996)) und bei der Behandlung viraler, bakterieller und parasitärer Infektionen sowie Neoplasien gesehen.

Die WO 94/05303 betrifft Peptid-Diagnostika und Therapeutika für Spondylarthropathien einschließlich HLA-B27-assoziierter Uveitis.

In den Dokumenten WO 88/05784 und WO 93/17699 wird eine Verwendung der Peptide für die Diagnose und/oder Behandlung von Autoimmunerkrankungen nur allgemein, ohne jegliche Substantiierung, z.B. hinsichtlich bestimmter Autoimmunerkrankungen, erwähnt. Wie bereits weiter oben ausgeführt wurde, scheinen jedoch bei Autoimmunerkrankungen auf Ebene der zellulären Immunantwort die Aktivitäten CD4-positiver T-Lymphozyten, insbesondere Helfer-T-Lymphozyten, Suppressor-T-Lymphozyten, Zytokine, und andere Mechanismen gegenüber der Aktivität von cytotoxischen T-Lymphozyten von größerer Bedeutung zu sein, so daß für die Behandlung von Autoimmunerkrankungen ein anderes, wahrscheinlich wesentlich breiteres Wirkungsspektrum der dafür vorgesehenen Peptide erforderlich ist.

Die Verwendung von Peptiden anstelle vollständiger Antigenproteine oder davon abgeleiteter langkettiger Fragmente empfiehlt sich aus einer Reihe von Gründen, da insbesondere bei einer geplanten Verabreichung an Menschen oder Tiere der Einsatz derartiger vollständiger Antigenproteine oder davon abgeleiteter langkettiger Fragmente eine Reihe von Schwierigkeiten bedingt:
1) Die sogenannte "Prozessierung", d.h. das Herausschneiden genau des immunologisch aktiven Abschnitts (Epitops), im Empfängerorganismus ist nicht immer absolut gewährleistet.
2) Das Antigen kann allenfalls mit großem Aufwand "gentechnologisch" synthetisiert werden.
3) Das Antigen wird dementsprechend im Allgemeinen aus natürlichem Gewebe isoliert werden. Hierbei kommt es leicht zu einer Infektion durch Bakterien, Viren und/oder Virione und ggf. zu einer nachfolgenden Transfektion durch (retro)virale oder bakterielle DNA.
4) Die Antigenproteine sind auch als Trockensubstanz unter Umständen nur sehr kurz lagerfähig und erfordern zudem besondere und aufwendige Lagerbedingungen.
5) Die Gefahr einer Allergisierung im Sinne einer Lebensmitteloder Kontaktallergie ist bei der Anwendung von Antigenproteinen am Menschen oder Tier ganz besonders zu erwarten, da es leicht zu einer Vernetzungswirkung von IgE auf Zelloberflächen von Mastzellen und Eosinophilen kommt.

Wie bereits angesprochen, wird die der Erfindung zugrundeliegende Aufgabe durch die erfindungsgemäße Verwendung bestimmter, von den α1- oder α2-Domänen von HLA-B27 bzw. humanem Keratin VI abgeleiteter Peptide zur Diagnose und Behandlung von HLA-abhängigen Autoimmunerkrankungen gelöst.

Gegenstand der Erfindung ist somit die Verwendung eines oder mehrerer Peptide mit einer der folgenden allgemeinen Sequenzen zur Bereitstellung/Herstellung eines Mittels zur Diagnose und/oder Behandlung von HLA-abhängigen Autoimmunkrankheiten:

BL₁-X-BL₂-Y-BL₃-Z

oder

BL₁-X-BL₂-Y-BL₃-Z-BL₄-W-BL₅

wobei BL₁ für 1 bis 2 Aminosäuren steht,
X für Asp steht,
BL₂ für 4 bis 5 Aminosäuren steht,
Y für Ile steht,
BL₃ für 3 bis 4 Aminosäuren steht,
Z für Lys-Ala-Gln oder Lys-Ala-Glu steht,
BL₄ für 6 bis 8 Aminosäuren steht,
W für Arg steht,
Bl₅ für 1 bis 2 Aminosäuren steht,
wobei ggf. C und/oder N-terminal weitere Aminosäurereste, Glykosylreste, Fettsäurereste oder Alkyl/Alkenylreste (C₁-C₁₆) vorhanden sind.

Ein weiterer Gegenstand der Erfindung ist die Verwendung eines oder mehrerer Peptide mit der folgenden Sequenz zur Bereitstellung/Herstellung eines Mittels zur Diagnose und/oder Behandlung von HLA-abhängigen Autoimmunkrankheiten:

BL₆-O-BL₇-M-BL₈

wobei BL₆ für 1 Aminosäure steht,
O für Leu-Arg steht,
BL₇ für 1 Aminosäure steht,
M für Tyr steht und
BL₈ für 0 bis 5 Aminosäuren steht,
wobei ggf. C und/oder N-terminal Glykosylreste, Fettsäurereste oder Alkyl/Alkenylreste (C₁-C₁₆) vorhanden sind.

Weitere Gegenstände der Erfindung werden in den abhängigen und unabhängigen Ansprüchen definiert. So betrifft die Erfindung auch ein Kit zur Verwendung in einem Assay zur Diagnose von HLA-abhängigen Autoimmunerkrankungen, der neben anderen, für den Assay benötigten Reagenzien eines oder mehrere der Peptide, wie sie in den Ansprüchen 1 bis 7 definiert sind, umfaßt.

Die hier in Betracht kommenden Autoimmunerkrankungen umfassen HLA-Klasse I- und -II-assoziierte Autoimmunerkrankungen, insbesondere, aber nicht ausschließlich, Erkrankungen des rheumatischen Formenkreises, wie Arthritiden, rheumatoide und reaktive Arthritis, Arthritis psoriatica und juvenile rheumatoide Arthritis, sowie Iritis, Psoriasis und Uveitis. Ferner sind HLA-B27-assoziierte Autoimmunerkrankungen zu nennen, zu deren Diagnose und Therapie die vorstehend definierten Peptide der Erfindung gleichfalls eingesetzt werden können. Eine Assoziation mit dem HLA-B27-Antigen besteht beispielsweise bei ankylosierender Spondylitis (Morbus Bechterew) sowie bei bestimmten Formen der vorstehend genannten Erkrankungen des rheumatischen Formenkrei-ses, insbesondere bei bestimmten Formen der juvenilen rheumatoiden Arthritis und Arthritis psoriatica, und bei besonderen Formen von Iritis, Uveitis und Psoriasis. Es ist festzuhalten, daß sich die Eignung der vorstehend definierten Peptide nicht auf die Diagnose und Behandlung bzw. Verhütung von HLA-B27-assoziierten Autoimmunerkrankungen beschränkt. Sie erstreckt sich vielmehr auch auf solche, die eine Assoziation mit anderen HLA-Antigenen der Klasse I zeigen, wie beispielsweise die nicht-HLA-B27-assoziierte juvenile rheumatoide Arthritis und Arthritis psoriatica. Die Eignung erstreckt sich ferner auch auf HLA-Klasse II-assoziierte Autoimmunerkrankungen, wie spezielle mit HLA-Klasse II-assoziierte Arthitiden.

Die erfindungsgemäß einsetzbaren Peptide umfassen von den α1-oder α2-Domänen von HLA-B27 bzw. humanem Keratin VI abgeleitete Peptide und, genauer, die nachfolgenden Kernsequenzen B27PA, B27PA*, B27PB, B27PC und Ker333 bzw. sind von diesen Kernsequenzen abgeleitet

Peptid B27PA:
Trp-Asp-Arg-Glu-Thr-Gln-Ile-Cys-Lys-Ala-Lys-Ala-Gln-Thr-Asp-
Arg-Glu-Asn-Leu-Arg-Thr,

Peptid B27PA*:
Trp-Asp-Arg-Glu-Thr-Gln-Ile-Cys-Lys-Ala-Lys-Ala-Gln,

Peptid B27PB:
Leu-Leu-Arg-Gly-Tyr-His-Gln-Asp-Ala-Tyr

Peptid B27PC:
Arg-Val-Ala-Glu-Gln-Leu-Arg-Ala-Tyr-Leu-Glu-Gly-Glu-Cys-Val

Peptid Ker333:
Leu-Asp-Leu-Asp-Ser-Ile-Ile-Ala-Glu-Val-Lys-Ala-Gln-Tyr-Glu-
Glu-Ile-Ala-Asn-Arg-Ser

Entsprechend der für HLA-B27 von Szöts, H., Riethmüller, G., Weiss, E. und Meo, T., in Proc. Natl. Acad. Sci. USA 83, 1428-1432 (1986), angegebenen Aminosäuresequenz entspricht
- das Peptid B27PA den Aminosäuren 60 - 80 innerhalb der α1-Domäne von HLA-B27,
- das Peptid B27PA* den Aminosäuren 60 - 72 innerhalb der α1-Domäne von HLA-B27,
- das Peptid B27PB den Aminosäuren 109 - 118 innerhalb der α2-Domäne von HLA-B27 und
- das Peptid B27PC den Aminosäuren 151 - 165 innerhalb der α2-Domäne von HLA-B27.

Die Erfinder haben gefunden, daß für die Aktivität der Peptide zur Verhütung bzw. Behandlung von HLA-abhängigen Autoimmunerkrankungen bzw. für die Diagnose derselben nur eine begrenzte Anzahl von Aminosäuren, die innerhalb der genannten Kernsequenzen diskontinuierlich verteilt sind, essenziell ist. Folglich können hinsichtlich der diesbezüglich nicht wesentlichen Aminosäurepositionen weitreichende Deletionen, Insertionen, Inversionen und Substitutionen vorgenommen werden, ohne die genannte biologische Wirksamkeit der Peptide zu beeinträchtigen. Selbstverständlich werden hierbei auch Derivatisierungen und Modifikationen der zugrundliegenden Aminosäuren mitumfaßt (Methylierung, Amidierung, Glykosylierung, Anhängen von Fettsäureresten (gesättigten und ungesättigten aliphatischen Resten).

Wesentlich bei jeglicher Abänderung von Peptiden gegenüber den Kernsequenzen ist, daß das resultierende Peptid eine *räumliche* oder *dreidimensionale Struktur* aufweist, die sich nicht aufgrund der durch die vorstehend definierte Sequenz sich ergebenden *Ladung und*/*oder Oberflächenstruktur* hinsichtlich des Verwendungszwecks als nachteilig erweist, und bevorzugt in der Weise, daß die räumliche Struktur des abgeänderten Peptids in ihrer *Oberflächenstruktur* und *Ladungsverteilung* derjenigen der vorstehend genannten Kernpeptide oder zumindest derjenigen eines für die biologische Wirksamkeit essenziellen Teilabschnitts der Kernpeptide entspricht.

Folglich lassen sich erfindungsgemäß einsetzbare, von B27PA, B27PA* und Ker333 abgeleitete Peptide durch die folgenden Formeln veranschaulichen:

BL₁-X-BL₂-Y-BL₃-Z-BL₄-W-BL₅

und

BL₁-X-BL₂-Y-BL₃-Z

Die Abkürzung BL steht für Aminosäureblöcke, die nach Maßgabe der Angaben in der nachfolgenden Tabelle 1 bis 8 Aminosäuren umfassen können. Zwischen diesen Blöcken finden sich einzelne charakteristische und möglicherweise für die vorstehend angesprochene biologische Wirksamkeit wesentliche Aminosäuren oder Aminosäurengruppen (X, Y, Z und W). Die Peptide B27PA und Ker333 wurden dementsprechend aufgrund der Übereinstimmungen in den Aminosäurepositionen X, Y, Z und W aus den jeweiligen Gesamtsequenzen ausgewählt.

Bezogen auf die vorstehend angegebenen Kernsequenzen von B27PA bzw. B27PA*
- entspricht X (an Position 2 des Peptids; Position 61 nach Szöts et al. (a.a.O.)) der Aminosäure Asparaginsäure (Asp),
- entspricht Y (an Position 7 des Peptids; Position 66 nach Szöts et al. (a.a.O.)) der Aminosäure Isoleucin (Ile),
- entspricht Z (Positionen 11 - 13 des Peptids; Positionen 70 - 72 nach Szöts et al. (a.a.O.)) der Tripeptidsequenz Lys-Ala-Gln, alternativ auch Lys-Ala-Glu,
- entspricht W (an Position 20 des Peptids; Position 79 nach Szöts et al. (a.a.O.)) der Aminosäure Arginin (Arg).

Bezogen auf die vorstehend angegebene Kernsequenz von Ker333 entsprechen die Reste X, Y, Z und W den gleichen Aminosäuren wie bei den von B27PA und B27PA* abgeleiteten Peptiden, entspricht also
- X (an Position 2 des Peptids) der Aminosäure Asparaginsäure (Asp),
- Y (an Position 7 des Peptids) der Aminosäure Isoleucin (Ile),
- Z (Positionen 11 - 13 des Peptids) der Tripeptidsequenz Lys-Ala-Gln, alternativ auch Lys-Ala-Glu,
- W (an Position 20 des Peptids) der Aminosäure Arginin (Arg).

Die in der im Folgenden aufgeführten Tabelle für die Blocksequenzen BL angegebenen Aminosäuren und Peptide entsprechen den in den Peptiden B27PA, B27PA* und Ker333 an diesen Positionen vorkommenden Aminosäuren; so ist BL₁ ein Block aus 1 bis 2 Aminosäuren, z.B. Trp oder Leu, wie sie in den Peptiden B27PA, B27PA* bzw. Ker333 an dieser Position vorkommen. Die Bezeichnung BL₂ umfaßt einen Block von 4 bis 5 Aminosäuren. Für beide Blöcke sind weitreichende Inversionen, Modifikationen und Substitutionen denkbar. Das gleiche gilt entsprechend für die Peptidblöcke BL₃ bis BL₅ mit 3 bis 4, 6 bis 8 bzw. 1 bis 2 Aminosäuren. Bevorzugt sind jedoch auch in diesem Falle Substitutionen durch homologe Aminosäuren gegenüber den Kernsequenzen B27PA, B27PA* und Ker333, obgleich bei den BL-Positionen auch weitergehende Substitutionen unter Aufrechterhaltung der gewünschten biologischen Aktivität möglich sein sollten.

Wie bereits wiederholt angesprochen, ist letztendlich entscheidend, daß eine *räumliche Struktur* entsteht, die sich nicht aufgrund der durch die vorstehend definierte Sequenz sich ergebenden *Ladung und*/*oder Oberflächenstruktur* hinsichtlich des Verwendungszwecks als nachteilig erweist, und bevorzugt in der Weise, daß die *räumliche Struktur* des abgewandelten Peptids insgesamt oder zumindest hinsichtlich eines mindestens 6, vorzugsweise mindestens 8 Aminosäuren langen Abschnitts in ihrer *Oberflächenstruktur* und *Ladungsverteilung* derjenigen der vorstehend genannten Kernpeptide oder eines entsprechenden Abschnitts davon im wesentlichen entspricht.

| BL₁ | X | BL₂ | Y | BL₃ | Z | BL₄ | W | BL₅ | Bezeichnung |
|---|---|---|---|---|---|---|---|---|---|
| 1-2 as | (2) | 4-5 as | (7) | 3-4 as | (11,12,13) | 6-8 as | (20) | 1-2 as | |
| | Asp | | Ile | | Lys-Ala-Gln oder Lys-Ala-Glu | | Arg | | |
| Trp | " | Arg-Glu- | " | Cys-Lys- | " | Thr-Asp- | " | Thr | B27PA |
| | | Thr-Gln | | Ala | | Arg-Glu-Asn-Leu | | oder Ile | |
| | | | | | | | | | |
| Trp | " | Arg-Glu- | " | Cys-Lys- | " | - | - | - | B27PA* |
| | | Thr-Gln | | Ala | | | | | |
| Leu | " | Leu-Asp- | " | Ala-Glu- | " | Tyr-Glu- | " | Ser | Ker333 |
| | | Ser-Ile | | Val | | Glu-Ile-Ala-Asn | | | |

BL₄ kann in von der vorstehend angegebenen Kernsequenz B27PA abgeleiteten Peptiden speziell auch für eine der folgenden Aminosäuregruppen stehen: Thr-Asp-Arg-Glu-Asp-Leu oder Thr-Asp-Arg-Glu-Ser-Leu oder Thr-Tyr-Arg-Glu-Asn-Leu oder Thr-Tyr-Arg-Glu-Asp-Leu oder Thr-Tyr-Arg-Glu-Ser-Leu. Ähnliches gilt für von den vorstehend angegebenen Kernsequenzen B27PB und B27PC abgeleitete Peptide mit der Sequenz:

BL₆-O-BL₇-M-BL₈.

Die Peptide B27PB und B27PC weisen übereinstimmend die als O und M bezeichneten Peptidpositionen auf.

Bezogen auf die vorstehend angegebene Kernsequenz B27PB
- entspricht O (Positionen 2 - 3 des Peptids; Positionen 110 - 111 nach Szöts et al. (a.a.O.)) der Dipeptidsequenz Leu-Arg und
- entspricht M (an Position 5 des Peptids; an Position 113 nach Szöts et al. (a.a.O.)) der Aminosäure Tyrosin (Tyr).

Bezogen auf die vorstehend angegebene Kernsequenz B27PC
- entspricht O (Positionen 6 - 7 des Peptids; Positionen 156 - 157 nach Szöts et al. (a.a.O.)) gleichfalls der Dipeptidsequenz Leu-Arg und
- entspricht M (an Position 9 des Peptids; an Position 159 nach Szöts et al. (a.a.O.)) gleichfalls der Aminosäure Tyrosin (Tyr).

Auch hier sollten im Falle einer Substitution die Positionen BL vorzugsweise durch homologe (d.h. strukturell und chemisch eng verwandte) Aminosäuren bzw. Aminosäurengruppen substituiert sein. Auch hier gilt, daß Substitutionen, Modifikationen, Inversionen, Deletionen und Insertionen von Aminosäuren in dem Umfange möglich sind, soweit diese Abänderungen nicht auf die durch die vorstehend definierte Sequenz sich ergebende *Ladung und*/*oder Oberflächenstruktur* hinsichtlich des Verwendungszwecks nachteilig einwirken, und bevorzugt in der Weise, daß dadurch eine räumliche Struktur hinsichtlich Oberflächenstruktur und Ladungsverteilung entsteht, die zumindest teilweise, d.h. zumindest über einen mindestens 6, vorzugsweise mindestens 8 Aminosäure langen Abschnitt, der für die biologische Aktivität des Peptids essenziell ist, hinweg, derjenigen der zugrundeliegenden Kernsequenz entspricht.

Die nachfolgende Tabelle gibt lediglich beispielhaft die entsprechenden Aminosäuren bzw. Peptidsequenzen an, die in den zugrundeliegenden Kernsequenzen B27PB und B27PC an diesen Positionen gefunden werden.

| Bezeichnung | BL₆ | O | BL₇ | M | BL₈ |
|---|---|---|---|---|---|
| | 1-5 as | 2 as | 1 as | 1 as | 0-6 as |
| | | Leu-Arg | | Tyr | |
| B27PB | Leu | " | Gly | " | His-Gln-Asp-Ala-Tyr |
| B27PC | Arg-Val-Ala-Glu-Gln | " | Ala | " | Leu-Glu-Gly-Glu-Cys-Val |

Es ist jedoch festzuhalten, daß Peptide, die jeweils unter eine der vorstehend angegebenen allgemeinen Formeln fallen, nicht notwendigerweise dasselbe Wirkungsspektrum aufweisen, was z.B. die HLA-abhängige Autoimmunkrankheit angeht, die durch diese Peptide behandelt werden kann. Abwandlungen in einzelnen oder nur wenigen Aminosäuren können bereits dazu führen, daß ein Peptid erfolgreich für die Behandlung einer gegenüber dem Ausgangspeptid anderen, HLA-abhängigen Autoimmunerkrankung, wie Iritis, Psoriasis, rheumatischen Erkrankungen, wie Morbus Bechterew (ankylosierende Spondylitis), Psoriasisarthritis (Arthritis psoriatica), juveniler rheumatoider Arthritis bei Knaben, einsetzbar ist.

Erfindungsgemäße Peptide im Sinne der oben beschriebenen diagnostischen oder therapeutischen Anwendungen sind auch solche Peptide, die 8 bis 20, bevorzugt 10 bis 18, optimal 12 bis 16, Aminosäuren aus den oben gezeigten Sequenzen umfassen.

Die Peptide im Sinne der Erfindung können auch N-terminal und/oder C-terminal Substitutionen aufweisen; hierzu können beispielsweise zählen:
Fettsäuren und Phospholipide,
Zuckerreste und
C₁-C₁₂-Alkyl- oder -Alkenylreste.

Die hierzu erforderlichen Kopplungsreagenzien und -verfahren sind dem Fachmann bekannt. Beispielhaft sei hier die Verwendung von Methyldithiobenzoesäure (MDTB) für die Konjugation mit aktivierten Olefinen über eine NH₂-Gruppe eines Peptids und die Verwendung von bifunktionalen Verknüpfungsreagenzien, wie MBSE, Glutaraldehyd und Methyldithiobenzoesäure (MDTB) für die Kopplung mit Phospholipiden, wie Phosphatidylcholin oder Phosphatidylethanolamin, aufgeführt.

Ein Peptid im Sinne der Erfindung kann direkt oder über Substituenten oder Kopplungssysteme (z.B. Biotin/Avidin) an organische oder anorganische Trägermaterialien oder, wie nachfolgend noch weitergehend ausgeführt werden wird, direkt oder indirekt nachweisbare Markierungssubstanzen gebunden oder adsorbiert sein.

Bei den vorstehend definierten Peptiden können ohne wesentlichen Wirkungsverlust verschiedene Änderungen vorgenommen werden, unter anderem:
1) Die Peptide können sowohl am N-terminalen als auch am C-terminalen Ende verlängert werden.
2) Die Peptide können sowohl am N-terminalen als auch am C-terminalen Ende verkürzt werden, jedoch in der Regel lediglich nur bis zu Peptiden mit mindestens 6, vorzugsweise mindestens 8 Aminosäuren.

Insbesondere im Falle der von B27PA und Ker333 abgeleiteten Peptide ist eine N- wie C-terminale Verkürzung auch über einzelne der charakteristischen Aminosäuren hinaus möglich, ohne daß die vorstehend definierte biologische Aktivität verloren geht. Ein Beispiel für ein C-terminal verkürztes, von B27PA abgeleitetes Peptid ist B27PA*, das, wie vorstehend dargestellt, die Aminosäuren 60 - 72 gemäß der Aminosäuresequenz von HLA-B27 nach Szöts et al. (a.a.O.) umfaßt.

Die Erfindung umfaßt gleichfalls die folgenden erfindungsgemäß einsetzbaren Peptide:

Peptid B27PA:
Trp-Asp-Arg-Glu-Thr-Gln-Ile-Cys-Lys-Ala-Lys-Ala-Gln-Thr-Asp-
Arg-Glu-Asn-Leu-Arg-Thr,

Peptid B27PA*:
Trp-Asp-Arg-Glu-Thr-Gln-Ile-Cys-Lys-Ala-Lys-Ala-Gln,

Peptid B27PB:
Leu-Leu-Arg-Gly-Tyr-His-Gln-Asp-Ala-Tyr,

Peptid B27PC:
Arg-Val-Ala-Glu-Gln-Leu-Arg-Ala-Tyr-Leu-Glu-Gly-Glu-Cys-Val,

Peptid Ker333:
Leu-Asp-Leu-Asp-Ser-Ile-Ile-Ala-Glu-Val-Lys-Ala-Gln-Tyr-Glu-
Glu-Ile-Ala-Asn-Arg-Ser.

Gegenstand der Erfindung sind ferner von den Sequenzen der vorstehend angegebenen Peptide abgeleitete Peptide, die eine vergleichbare Eignung zur Diagnose und/oder eine vergleichbare Aktivität bei der Behandlung von HLA-abhängigen Autoimmunerkrankung aufweisen. Als vergleichbare Aktivität werden im Rahmen der vorliegenden Anmeldung eine bis zu 200% höhere, im Einzelfall bis zu 300% höhere, oder eine bis zu 25% niedrigere Aktivität angesehen. Die erfindungsgemäßen, von den vorstehend angegebenen Sequenzen abgeleiteten Peptide sind von diesen insbesondere durch Substitution einzelner Aminosäuren oder kleineren Gruppen von Aminosäuren abgeleitet. Bevorzugt sind diese dergestalt abgeleitet, daß
- eine geringe Anzahl, in der Regel nicht mehr als vier oder fünf der Aminosäuren, einzeln oder in kleinen Gruppen durch eine oder mehrere vorzugsweise homologe Aminosäure(n) substituiert ist und zusätzlich/oder
- eine geringe Anzahl, in der Regel nicht mehr als bis zu 3 beliebige Aminosäurereste N- und/oder C-terminal angefügt sind und/oder
- die Peptide N- und/oder C-terminal um eine geringe Anzahl von Aminosäuren verkürzt sind; die Verkürzung geht in der Regel lediglich so weit, daß ein Peptid mit mindestens 6, vorzugsweise mindestens 8 Aminosäuren resultiert.

Wie bereits ausgeführt, wurden die Peptide B27PA und Ker333 aufgrund von Sequenzhomologien an bestimmten Aminosäurepositionen aus den jeweiligen Gesamtsequenzen ausgewählt. Es wird angenommen, daß diese Aminosäurepositionen zumindest zum Teil für die biologische Wirksamkeit der Peptide von Bedeutung sein könnten. Die Sequenzhomologien sind in den folgenden Kernsequenzen B27PA, Ker333 und der von B27PA durch C-terminale Verkürzung abgeleiteten Kernsequenz B27PA* durch Unterstreichung der einzelnen Aminosäuren angegeben:

Peptid B27PA:
Trp-Asp-Arg-Glu-Thr-Gln-Ile-Cys-Lys-Ala-Lys-Ala-Gln-Thr-Asp-
Arg-Glu-Asn-Leu-Arg-Thr,

Peptid Ker333:
Leu-Asp-Leu-Asp-Ser-Ile-Ile-Ala-Glu-Val-Lys-Ala-Gln-Tyr-Glu-
Glu-Ile-Ala-Asn-Arg-Ser,

Peptid B27PA*:
Trp-Asp-Arg-Glu-Thr-Gln-Ile-Cys-Lys-Ala-Lys-Ala-Gln.

Unter den Maßgaben der vorstehend genannten vergleichbaren Eignung zur Diagnose und/oder vergleichbaren Aktivität bei der Behandlung von HLA-abhängigen Autoimmunerkrankung und Berücksichtigung der vorstehenden Ausführungen zu der räumlichen Struktur der Peptide sind gleichfalls Peptide von der Erfindung umfaßt, die, gegebenenfalls zusätzlich zu Substitutionen, durch Modifizierung (z.B. durch Methyl-, Fettsäure-, Zuckergruppen), Inversion, Deletion und/oder Insertion einzelner Aminosäuren oder kleinerer Gruppen von Aminosäuren von den vorstehend angegebenen Sequenzen abgeleitet sind.

Mögliche Strategien zur Erzeugung von von den vorstehend angegebenen Kernsequenzen abgewandelten Peptiden umfassen dementsprechend Methoden, die diese jeweils besondere räumliche Struktur der Kernsequenzen oder von Abschnitten von diesen berücksichtigen. Beispielhaft wären hier die bereits genannten homologen Aminosäureaustausche zu nennen, aber auch computergestütztes "molecular modelling" mit dem Ziel, festzustellen, in wie weit mögliche Aminosäuresubstituierungen, -modifizierungen, -deletionen oder -insertionen die räumliche Struktur des resultierenden Peptids beeinflussen. Eine von den "natürlichen" Aminosäurebausteinen losgelöste Alternative bietet das Konzept der "Peptidomimetics" mit dem Ziel, Nicht-Peptid-Analoga zu erzeugen, die jedoch eine dreidimensionale Struktur ausbilden, die der eines gegebenen Ausgangspeptids, hier der vorstehend genannten Kernsequenzen oder von Abschnitten davon, gleicht.

Die der vorstehenden Definition entsprechenden Peptide können gleichfalls daran gebundene Markierungssubstanzen, wie sie weiter oben angesprochen wurden, umfassen, sofern diese die Eignung der Peptide zur Diagnose von HLA-abhängigen Autoimmunerkrankungen nicht wesentlich beeinträchtigen.

Die erfindungsgemäß einsetzbaren bzw. erfindungsgemäßen Peptide können auf eine Vielzahl von Wegen hergestellt werden. Beispielsweise kann eine chemische Synthese unter Einsatz automatisierter oder manuell zu bedienender Peptid-Synthetisiervorrichtungen, wie sie beispielsweise von den Firmen Beckman oder Applied Biosystems, Inc., vertrieben werden, erfolgen. In diesem Falle wird entsprechend den von den Herstellern dieser Apparate bereitgestellten Protokollen zur Peptidsynthese, die genaue Angaben zu den einzusetzenden Reagenzien und Verfahrensbedingungen enthalten, vorgegangen.

Weitere Möglichkeiten bestehen in einer manuellen chemischen Synthese. Unter den dem Fachmann bekannten Verfahren sei beispielhaft auf das Festphasenverfahren nach R. B. Merrifield ("Solid Phase Synthesis", Science 232, 341-347 (1986)), das in Lösung ausgeführte Verfahren unter Einsatz von Dicyclohexylcarbodiimid (DCCI) zur Aktivierung von Carboxylgruppen von Ausgangs-Aminoacylverbindungen oder -Peptidteilsequenzen der gewünschten Struktur und Umsetzung der so carboxyterminal aktivierten Verbindungen mit den Aminofunktionen entsprechender Verbindungen oder Fragmente, die sich in dem endgültigen Peptid an den entsprechenden Positionen befinden sollen, verwiesen. Im Falle des letztgenannten Verfahrens ist eine Blockierung von unter diesen Bedingungen gleichfalls reaktiven, zusätzlichen Gruppen innerhalb der Reaktionspartner erforderlich; für Aminogruppen hat sich beispielsweise der Einsatz von *tert.*-Butyloxycarbonyl-Gruppen (*t*-BOC-Gruppen) etabliert.

Die Peptide können auch aus natürlichen Quellen, insbesondere Bakterien oder Eukaryontenzellen, die mit einer die Peptide kodierenden DNA in einer Weise, die die Expression derselben ermöglicht, transformiert wurden, isoliert werden. Die hierfür erforderlichen Verfahren z.B. zur DNA-Manipulation, Transformation und Peptidgewinnung durch Aufreinigung z.B. mittels bekannter Chromatographieverfahren (Ionenaustausch- und Immunaffinitätschromatographie, Gelfiltration, ...) und Elektrophorese sind dem Fachmann bekannt. Derartige aus natürlichen Quellen gewonnene Peptide eignen sich insbesondere für den Einsatz zur Diagnose von HLA-abhängigen Autoimmunerkrankungen.

Wie bereits ausgeführt umfaßt die Erfindung insbesondere die Verwendung der genannten Peptide zur Diagnose und Behandlung von HLA-abhängigen Autoimmunerkrankungen.

### a) Diagnose von HLA-abhängigen Autoimmunerkrankungen

Die von HLA-B27 und Keratin VI abgeleiteten Peptide können diagnostisch für Nachweise zellulärer sowie humoraler Immunantworten eingesetzt werden. Dadurch können die Krankheitsbilder immunologisch genauer klassifiziert werden, was eine spezifischere Behandlung ermöglicht. Aufgrund der dargestellten Sequenzen stellen diese Peptide ein spezifischeres Antigen dar als das jeweilige vollständige Proteinantigen. Darüberhinaus stellt bei Keratin die Löslichkeit des Peptids im Gegensatz zum Protein einen erheblichen Anwendungsvorteil dar.

Die hier in Betracht kommenden Autoimmunerkrankungen umfassen HLA-Klasse I- und -II-assoziierte Autoimmunerkrankungen, insbesondere, aber nicht ausschließlich, Erkrankungen des rheumatischen Formenkreises, wie Arthritiden, rheumatoide und reaktive Arthritis, Arthritis psoriatica und juvenile rheumatoide Arthritis, sowie Iritis, Psoriasis und Uveitis. Ferner sind HLA-B27-assoziierte Autoimmunerkrankungen zu nennen, zu deren Diagnose und Therapie die vorstehend definierten Peptide dieser Erfindung eingesetzt werden können. Eine Assoziation mit dem HLA-B27-Antigen besteht bei ankylosierender Spondylitis (Morbus Bechterew) sowie bei bestimmten Formen der vorstehend genannten Erkrankungen des rheumatischen Formenkreises, insbesondere bei bestimmten Formen der juvenilen rheumatoiden Arthritis und Arthritis psoriatica, und bei besonderen Formen von Iritis, Uveitis und Psoriasis. Es ist festzuhalten, daß sich die Eignung der vorstehend definierten Peptide nicht auf die Diagnose und Behandlung bzw. Verhütung von HLA-B27-assoziierten Autoimmunerkrankungen beschränkt. Sie erstreckt sich vielmehr auch auf solche, die eine Assoziation mit anderen HLA-Antigenen der Klasse I zeigen, wie beispielsweise die nicht-HLA-B27-assoziierte juvenile rheumatoide Arthritis und Arthritis psoriatica. Die Eignung erstreckt sich ferner auch auf HLA-Klasse II-assoziierte Autoimmunerkrankungen, wie spezielle mit HLA-Klasse II-assoziierte Arthitiden.

Besonders eignen sich die Peptide als Antigene für in vitro-Nachweissysteme zur Messung von Antikörpern, wie z.B. im enzymgekoppelten Immunadsorptionstest (ELISA) oder Immunfluoreszenz-Test, und zur Messung der zellulären Immunantwort, wie z.B. im T-Zell-Stimulationstest.

Dabei können die Peptide direkt gelöst oder an Trägermaterial adsorbiert oder kovalent gebunden eingesetzt werden. Entsprechend dem Assayverfahren werden die Peptide mit einer direkt oder indirekt nachweisbaren Markersubstanz gekoppelt eingesetzt. Derartige dem Fachmann bekannte Markersubstanzen umfassen u.a. direkt oder über Kopplungssysteme (z.B. Biotin/Avidin) gebundene fluoreszierende Stoffe, Enzyme, radioaktive Elemente (³H, ¹³C, ³²P, ³⁵S, ¹²⁵I, ...) umfassende Gruppierungen oder Moleküle und magnetische Teilchen. Die Peptide können dazu in freier oder entsprechend modifizierter Form mit weiteren für die Durchführung des Assayverfahrens benötigten Reagenzien in Form eines Kits bereitgestellt werden.

Ebenso können die Peptide auch direkt am Menschen zu diagnostischen Zwecken verwendet werden. Hierbei kann durch die intra- oder subkutane Injektion des Peptids - in Lösung oder an einen Trägerstoff adsorbiert oder gebunden - eine immunologische Reaktion ausgelöst und abgelesen werden. Insbesondere eignen sich die Peptide für die Auslösung einer Immunreaktion vom verzögerten Typ bei gesunden Menschen als diagnostische Suchmethode oder beim Patienten zur spezifischen Diagnostik. Hierfür werden die Peptide in Form von Formulierungen, die ggf. für eine derartige Verwendung dem Fachmann bekannte Zusatzoder Hilfsstoffe umfassen, auf üblichem Weg, z.B. oral, intravenös, subkutan, intramuskulär u.s.w., verabreicht.

### b) Verhütung/Therapie von HLA-abhängigen Autoimmunerkrankungen

Die vorstehend definierten, von HLA-B27 und Keratin VI abgeleiteten Peptide können als Therapeutika für HLA-abhängige Autoimmunerkrankungen eingesetzt werden, wie sie vorstehend unter a) beispielhaft aufgeführt wurden.

Ein weiterer Gegenstand der Erfindung ist dementsprechend die Verwendung der erfindungsgemäßen Peptide zur Verhütung oder Behandlung von HLA-abhängigen Autoimmunerkrankungen und insbesondere für die Herstellung von Mitteln zur Verhütung und/oder Behandlung von HLA-abhängigen Autoimmunerkrankungen.

Die Erfindung umfaßt auch ein Verfahren zur Behandlung von HLA-abhängigen Autoimmunerkrankungen beim Menschen, das eine Verabreichung der vorstehend definierten Peptide umfaßt.

Ziel der erfindungsgemäßen Verabreichung der genannten Peptide ist eine Tolerisierung gegenüber verschiedenen Autoantigenen, die Ziel der immunologischen Reaktionen sind, gegen die sich die autoimmune Aktivität richtet. Dabei lösen die Peptide eine spezifische Immunreaktion aus, die sich als Toleranz, Anergisierung oder Deletion von antigenspezifischen T-Zellen äußert. Eine Toleranzinduktion kann aber auch durch die Blockade der antigenpräsentierenden Restriktionselemente auf professionellen oder nicht-professionellen antigenpräsentierenden Zellen erreicht werden.

Wie bereits ausgeführt, ist die Reaktion des Immunsystems bei derartigen Autoimmunerkrankungen nicht auf Blutlymphozyten beschränkt. Es wird erwartet, daß die humorale Immunantwort über Antikörper, Makrophagen und weitere Zellen des Immunsystems ebenfalls mögliche Ziele der erfindungsgemäßen Peptide umfaßt.

Der Nachweis des therapeutischen Potentials der Peptide ergibt sich aus experimentellen Befunden bei Patienten und Normalspendern. Dabei wurden beispielhaft zum einen in vitro Untersuchungen durchgeführt, bei denen die Lymphozyten aus dem peripheren Blut (PBL) von Patienten mit Morbus Bechterew (ankylosierender Spondylitis) und gesunden Spendern gewonnen wurden. Diese Lymphozyten wurden dann mit verschiedenen Proteinen und Peptiden inkubiert und die Reaktion als verstärkte Zellvermehrung mittels Einbau von ³H-Thymidin gemessen. Dabei zeigte sich, daß die Lymphozyten von Patienten mit Morbus Bechterew im Gegensatz zu Gesunden deutlich stärker auf das Peptid B27PA* reagierten als auf ein Vergleichspeptid (B27PD), das nicht den vorstehend angegebenen Definitionen entsprach. Aus der Primärstruktur der Peptide (Aminosäuresequenz) kann eine Kreuzreaktivität der Lymphozyten abgeleitet werden, die die Ursache der Autoimmunreaktion ist.

Darüberhinaus wurden Immunisierungsversuche mit den Peptiden B27PA* und Ker333 bei Ratten vorgenommen, die in einem von vier bzw. in zwei von vier Fällen zu geschwollenen Fingergelenken bei den Tieren führten, die den Befunden bei Psoriasisarthritis (Arthritis psoriatica) vergleichbar waren. Diese Befunde sind gleichfalls ein Hinweis darauf, daß die Peptide bei den Tieren, die eine Reaktion zeigten, eine Kreuzreaktivität mit natürlich vorkommenden Epitopen im Bereich der Fingergelenke aufweisen könnten, die die Ursache der Autoimmunreaktion ist. Können derartige Befunde erzielt werden, erscheint es sinnvoll, die entsprechenden Peptide bei Verfahren zur Diagnose bzw. weitergehenden Charakterisierung der jeweiligen Autoimmunerkrankung, wie sie beispielsweise im vorangehenden Absatz beschrieben worden sind, miteinzubeziehen.

Schon früher konnte im Falle der Autoimmun-Uveitis gezeigt werden, daß sich aus einer solchen Kreuzreaktivität unmittelbar ein therapeutischer Ansatz ergibt (siehe z.B. WO 95/29194; Thurau, S.R., et al., Immunology Letters 57, 193-201 (1997)). Für die Behandlung von Patienten ist daher die Kenntnis der immunologischen Reaktivität eines Peptids diagnostisch wichtig, da sich daraus unmittelbar eine Therapiemöglichkeit mit eben diesen Peptiden ergibt, indem sie zur Abschwächung einer autoaggressiven Immunantwort eingesetzt werden sollen.

Die Toleranz kann dann durch die intravenöse Gabe, subkutane oder intramuskuläre Verabreichung, aber auch insbesondere durch Aufbringung der vorstehend definierten Peptide bzw. diese enthaltender Präparate auf mukosale Oberflächen, wie z.B. des Nasopharynx-Bereichs, der Lunge oder des Magen-Darmtrakts (insbesondere bei oraler Verabreichung), ausgelöst werden.

Es wird in diesem Zusammenhang eine Verabreichung sowohl von einzelnen als auch von zwei oder mehreren der vorstehend definierten Peptide in Betracht gezogen. Bei der Verabreichung zweier oder mehrerer Peptide besteht die Möglichkeit einer gleichzeitigen oder einer sequenziellen Verabreichung.

Die die genannten Peptide enthaltenden Präparate können als Trockensubstanz, in Lösung, als Suspension oder auch als Aerosole, z.B. in Form von Nasensprays oder Inhalationssprays, verabreicht werden. Sie enthalten dann neben der Wirksubstanz übliche Hilfs- und Trägerstoffe. Die Peptide sollten in einer Tagesdosis von 10 Mikrogramm bis 1000 Milligramm pro Kilogramm Körpergewicht verabreicht werden.

Eine erfindungsgemäß besonders bevorzugte Verabreichungsweise erfolgt über Schleimhäute, insbesondere die Nasen-, Lungen- und Magen-Darm-Schleimhäute mittels eines Nasen- oder Inhalationssprays bzw. durch eine orale Verabreichung des/der Peptide. Die Mechanismen der oralen, nasalen oder Inhalationstoleranz-Induktion sind noch weitgehend unbekannt. Nach den derzeitigen Kenntnissen spielen wohl cytotoxische Effekte keine Rolle, zumindest nicht über CTL. Die regulatorischen Zellen, die durch mukosale Antigenverabreichung induziert werden, sind bislang weder ausreichend identifiziert noch funktionell charakterisiert.

Für eine orale Verabreichung kann eine Verwendung von Abgabesystemen, z.B. Kapseln, in Betracht kommen, aus denen die Peptide erst in spezifischen Kompartimenten des Gastrointestinaltrakts, z.B. in speziellen Darmabschnitten, und gegebenenfalls über einen längeren Zeitraum kontinuierlich freigesetzt werden.

Dadurch kann beispielsweise ein Kontakt des Präparats mit dem im Magen vorherrschenden aggressiven, sauren Milieu vermieden werden. Für eine subkutane Verabreichung stehen gleichfalls Abgabevorrichtungen zur Verfügung, die in diesem Falle implantiert werden. Abgabevorrichtungen der vorstehend angesprochenen Typen sind dem Fachmann bekannt.

Eine topische Verabreichung der Peptide zu Therapiezwecken wird im vorliegenden Falle nicht ausgeschlossen, erscheint jedoch nach gegenwärtigem Wissenstand als nicht bevorzugt. Im Falle von Psoriasis beispielsweise könnten autoaggressive T-Lymphozyten in den Hautläsionen topisch verabreichtes Peptid als bereits geeignet aufbereitetes, d.h. proteolytisch fragmentiertes Antigen im entsprechenden MHC-Kontext erkennen und die autoaggressiven Reaktionen dadurch verstärkt werden.

Gegenüber der Toleranzinduktion mit einem kompletten Protein, wie sie zur Zeit in verschiedenen Zentren erprobt wird, weist die Toleranzinduktion mit einem der vorstehend genannten Peptide bei HLA-abhängigen Autoimmunerkrankungen verschiedene Vorteile auf:
1) Ein Peptid kann relativ günstig auf proteinchemischem Weg synthetisiert werden.
2) Ein Peptid wird nicht aus natürlichem Gewebe isoliert. Somit entfallen alle möglichen Risiken einer Infektion durch Viren, Virione oder Bakterien, von denen das Gewebe möglicherweise befallen sein könnte, bzw. ggf. nachfolgender Transfektion mit entsprechender (retro)viraler oder bakterieller DNA.
3) Ein aufwendiges und in der Ausbeute meist nur wenig ergiebiges gentechnologisches Produktionsverfahren, das alternativ zur Gewinnung von Proteinen aus tierischem Material genutzt werden kann, ist nicht erforderlich.
4) Peptide sind in der Regel als Trockensubstanz lange lagerfähig und erfordern keine besonderen Lagerbedingungen.
5) Die Gefahr einer Allergisierung im Sinne einer Lebensmitteloder Kontaktallergie ist bei Peptiden im Gegensatz zu Proteinen nicht zu erwarten, da keine Vernetzungswirkung von IgE auf Zelloberflächen von Mastzellen und Eosinophilen erzielt werden kann.
6) Der Effekt der Peptide ist, wie vorstehend geschildert, eine regulierende Wirkung auf das Immunsystem.

### BEISPIELE

Die erfindungsgemäße Eignung von den vorstehenden Definitionen entsprechenden Peptiden als Antigene für in vitro-Nachweissysteme zur Messung der zellulären Immunantwort wurde anhand des Peptids B27PA* durch diverse Untersuchungen gezeigt.

### Peptide:

Die dem Test unterzogenen Peptide waren das vorstehend angegebene, gegenüber der Kernsequenz B27PA um 8 Aminosäuren C-terminal verkürzte Peptid B27PA* mit den Aminosäuren 60 - 72 der HLA-B27-Sequenz nach Szöts et al. (a.a.O.): 
B27PA*:
Trp-Asp-Arg-Glu-Thr-Gln-Ile-Cys-Lys-Ala-Lys-Ala-Gln
 sowie das nicht den vorstehenden Maßgaben für erfindungsgemäß einsetzbare Peptide entsprechende Kontrollpeptid B27PD mit den Aminosäuren 125-138 der HLA-B27-Sequenz nach Szöts et al. (a.a.O.):

B27PD:
Ala-Leu-Asn-Glu-Asp-Leu-Ser-Ser-Trp-Thr-Ala-Ala-Asp-Thr.

Es ist bereits früher gezeigt worden, daß das Peptid B27PD im Rattenmodell für experimentelle Autoimmun-Uveitis (EAU) uveitogen war (WO 95/29194; Wildner, G. & Thurau, S.R., Eur. J. Immunol. 24, 2579-2585 (1994)).

Das Peptid B27PA* wurde auf einer halbautomatischen Peptid-Synthetisiervorrichtung nach den Anweisungen des Herstellers hergestellt. Das Peptid B27PD wurde von Neosystems, Strasburg, Frankreich, erhalten. Die Peptide wurde in unsupplementiertem RPMI-1640 gelöst.

### Patienten:

Es wurden T-Lymphozyten aus peripherem Blut (PBL) von HLA-B27⁺-Patienten mit ankylosierender Spondylitis (AS), HLA-B27⁺-Patienten mit anderen Spondylarthropathien, HLA-B27⁻-Patienten mit rheumatoider Arthritis (RA), gesunden HLA-B27⁺- und gesunden HLA-B27⁻-Kontrollpersonen untersucht.

*HLA-B27*^{*+*}*-Patienten mit ankylosierender Spondylitis (AS):*
Die in die Studie aufgenommenen 55 Patienten mit ankylosierender Spondylitis (AS) erfüllten sämtlich die modifizierten New York-Kriterien für eindeutige AS (Van der Linden, S., Valkenburg, H.A., Cats, A., Arthritis Rheum. 27, 361-368, (1984)): (36 Männer, 19 Frauen; Altersdurchschnitt: 40,9 Jahre, Altersbereich: 18 - 71 Jahre). Sämtliche Patienten erfüllten die Voraussetzungen für eine klinisch "aktive" Erkrankung.

*HLA-B27*^{*+*}*-Patienten mit anderen Spondylarthropathien:*
Die in die Studie aufgenommenen 28 HLA-B27⁺-Patienten mit anderen Spondylarthropathien umfaßten 5 Patienten mit reaktiver Arthritis, 10 Patienten mit Arthritis psoriatica und 13 Patienten mit Arthritis enteropathica in Verbindung mit Morbus Crohn oder ulcerativer Colitis (15 Männer, 13 Frauen; Altersdurchschnitt: 42,4 Jahre, Altersbereich: 22 - 76 Jahre).

*Gesunde HLA-B27*^{*+*}*-Kontrollpersonen*:
Es wurden 30 Personen in die Studie aufgenommen (18 Männer, 12 Frauen; Altersdurchschnitt: 46,2 Jahre, Altersbereich: 20 - 78 Jahre).

*Gesunde HLA-B27*^{*-*}*-Kontrollpersonen:*
Es wurden 22 Personen in die Studie aufgenommen (10 Männer, 12 Frauen; Altersdurchschnitt: 30,8 Jahre, Altersbereich: 22 - 53 Jahre).

### HLA-B27⁻-Patienten mit rheumatoider Arthritis (RA):

Es wurden 7 HLA-B27⁻-Patienten mit aktiver rheumatoider Arthritis (RA) in die Studie aufgenommen (1 Mann, 4 Frauen; Altersdurchschnitt: 56,8 Jahre, Altersbereich: 30 - 72 Jahre).

Keiner der Kontroll-Blutspender hatte eine persönliche oder familiäre Vorgeschichte von Spondylarthropathien noch gab es Hinweise für eine noch nicht lange zurückliegende Infektion im Gastrointestinal- oder Urogenitalbereich.

### Isolierung von Lymphozyten aus peripherem Blut (PBL):

Aus heparinisiertem peripherem Blut gewonnene PBL wurden durch Standardzentrifugation über einen Ficoll-Hypaque-Gradienten isoliert, dreimal gewaschen und in RH10-Kulturmedium [RPMI-1640-Medium, das 2 mM L-Glutamin, HEPES, 100 U/ml Penicillin, 100 µg/ml Streptomycin und 10% hitzeinaktiviertes humanes AB-Serum enthält] resuspendiert.

### Lymphozyten-Proliferationsassay:

5 x 10⁴ PBL wurden in Rundboden-Mikrotiterplatten mit 96 Vertiefungen (Nunc, Roskilde, Dänemark) mit oder ohne Stimulanzien auf ein Endvolumen von 100 µl mit Kulturmedium RH10 aufgefüllt. Die Peptide wurden in einer Endkonzentration von 10 µg/ml verwendet. Sämtliche Tests wurden als Dreifachproben ausgeführt. Die Kulturen wurden 6 Tage bei 37°C in befeuchteter Atmosphäre (5% CO₂, 95% Luft) inkubiert. Am Tag 5 wurden 0,25 µCi ³H-Thymidin (³H-TdR; Amersham, Braunschweig, DE) jeder Vertiefung zugesetzt. Am Tag 6 wurden die Zellen auf Glasfilterpapier durch Standard-Ernteverfahren gesammelt. Der ³H-TdR-Einbau wurde durch Flüssigkeitsszintillationszählung quantifiziert. Die Ergebnisse sind als Stimulationsindizes angegeben ([cpm mit Stimulans - cpm ohne Stimulans] : [cpm ohne Stimulans]).

### Ermittlung der Dosis-Reaktions-Kurve für das Peptid B27PA*:

1 x 10⁵ PBL von einem auf B27PA* ansprechenden Patienten (AS1) in 100 µl RH10-Kulturmedium wurden ohne und mit dem synthetischen Peptid B27PA* in unterschiedlichen Konzentrationen von 0,1 bis 50,0 µg/ml 6 Tage inkubiert, mit 0,25 µCi ³H-Thymidin (³H-TdR) am Tag 5 gepulst und am Tag 6 auf Glasfilterpapier geerntet. Die Ergebnisse sind als mittlere Counts pro min (cpm) ± Standardabweichung von Dreifachproben angegeben.

### Erzeugung B27PA*-spezifischer T-Zellinien:

3 x 10⁶ PBL wurden mit 10 µg/ml des Peptids B27PA* in Platten mit 12 Vertiefungen in einem Volumen von 2 ml Kulturmedium RH10 inkubiert. Die PBL wurden von den Patienten AS1 und AS3 mit ankosylierender Spondylitis und einem gesunden HLA-B27⁺-Blutspender H1 gewonnen, die signifikante Proliferationsantworten auf das Peptid B27PA* in dem vorangehenden Lymphozyten-Proliferationsassay gezeigt hatten (siehe "ERGEBNISSE"); PBL von einem weiteren Patienten mit ankylosierender Spondylitis, AS2, der keine Proliferationsantwort zeigte, wurden als Kontrolle herangezogen. Nach 3 Tagen Kultur wurde das Medium durch frisches RH10, das mit 20 U/ml rekombinantem IL-2 (rIL-2, Boehringer Ingelheim, DE) supplementiert war, ersetzt. Am Tag 10 wurden die Kulturen gewaschen und mit autologen, bestrahlten (4000 rad) PBL als Feeder-Zellen, 10 µg/ml B27PA*-Peptid und rIL-2-supplementiertem RH10 restimuliert. Für die Restimulierung wurden 2 x 10⁶ Feeder-Zellen je 1 x 10⁶ Responder-Zellen verwendet. Am Tag 20 wurden die resultierenden T-Zellinien funktional in einem Lymphozyten-Proliferationsassay unter Verwendung von 1 x 10⁴ T-Zellen als Responder-Zellen und 1 x 10⁴ autologen E-(d.h. Monozyten/B-Zell-angereicherten Fraktionen, die aus autologen PBL durch Rosettenbildung mit Schaferythrozyten abgetrennt worden waren) als Feeder-Zellen in einem Gesamtvolumen von 150 µl RH10 charakterisiert, wie beschrieben in Hermann, E., Fleischer, B., Mayet, W.-J., Poralla, T., Meyer zum Büschenfelde, K.-H., Clin. Exp. Immunol. 75, 365-370, (1989). Die Peptide B27PA* (in 10 oder 1 µg/ml) und B27PD (in 10 µg/ml) wurden als Stimulanzien, Phytohaemagglutinin (PHA; 1 µg/ml) als Positivkontrolle verwendet. Um die Fähigkeit monoklonaler Antikörper (MAbs) zu überprüfen, die Peptid-spezifische T-Zell-Proliferation zu blockieren, wurden die MAbs w6/32 (anti-HLA-Klasse I; 1:400), DA6.231 (anti-HLA-Klasse II; 1:400) bzw. 1 µg/ml TCR-δ1 (spezifisch für eine Framework-Determinante auf der δ-Kette von γδ-TCR; T-Cell Sciences, Cambridge, MA, USA) den B27PA* enthaltenden Kulturen zugesetzt. Die Zellen wurden mit 0,25 µCi ³H-TdR am Tag 3 der Kultur gepulst und, wie vorstehend beschrieben, geerntet und gezählt.

### Phänotypische Analyse (FACS) von Peptid-stimulierten T-Zellinien:

Eine Analyse der T-Zellinien wie auch von unstimulierten PBL in einer Zellsortierungsvorrichtung wurde an den Tagen 8 und 20 der Kultur ausgeführt. 3 x 10⁵ Zellen wurden pro Test zuerst mit den unkonjugierten MAbs anti-CD3 (OKT3), anti-CD4 (OKT 4), anti-CD8 (OKT 8) (Hybridom-Überstände), BMA.031 (spezifisch für eine Framework-Determinante auf TCR-αβ), TCR-δ1 und dann mit mit Fluoreszeinisothiocyanat konjugiertem polyklonalem Ziegeanti-Maus-IgG-Antikörper (Medac, Hamburg, DE) in RPMI-1640/10% fötales Kälberserum markiert. Die Proben wurden zwischen jedem Schritt zweimal gewaschen. Eine cytofluorometrische Analyse, bei der 6000 Zellen pro Probe gezählt wurden, wurde an einer Fluoreszenz-aktivierten Zellsortierungsvorrichtung (FACStar, Becton Dickinson) ausgeführt. Die Ergebnisse sind als Prozentsatz der durch monoklonale Antikörper (MAb) definierten, positiven, angefärbten Zellen bezogen auf den Prozentsatz aller CD3-positiven T-Zellen angegeben.

### ERGEBNISSE

### Proliferationsantworten von PBL auf von HLA-B27 abgeleitete Peptide:

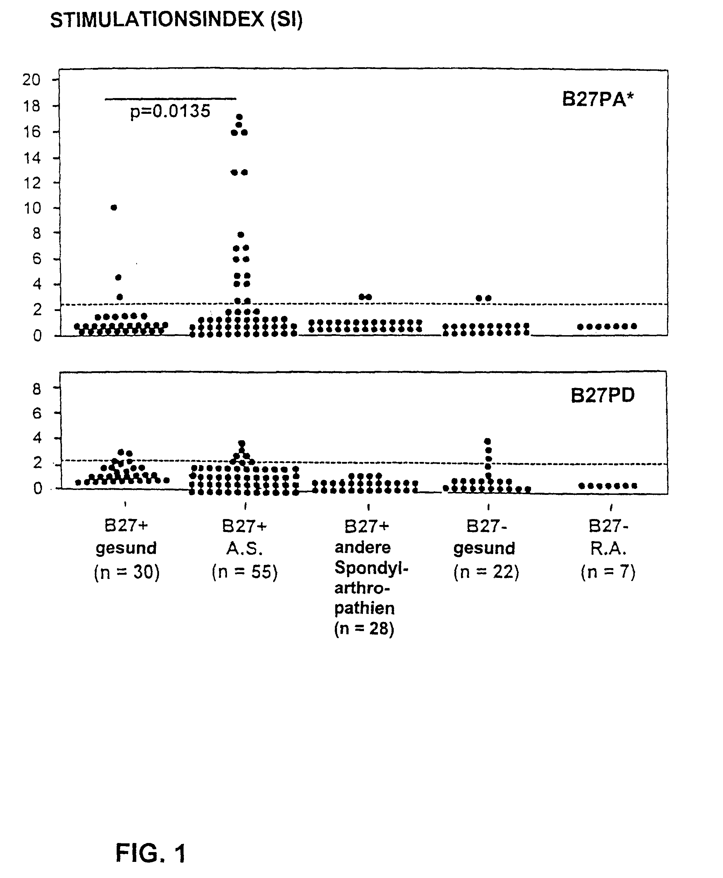

Wie in Figur 1 gezeigt, induzierte B27PA* eine signifikante PBL-Proliferation (SI ≥ 2,5) in 17/55 untersuchten Patienten mit AS (SI zwischen 2,5 und 17) wie auch in 3/30 gesunden HLA-B27⁺-Blutspendern (SI zwischen 2,5 und 9,8). Der Unterschied zwischen der Gruppe von AS-Patienten und der Gruppe von gesunden HLA-B27⁺-Blutspendern war statistisch signifikant (p = 0,0135; Mann-Whitney-Rangsummentest; Programm Stat Exact). Bei 2/28 anderen HLA-B27-assoziierten Spondylarthropathien wie auch bei 2/22 gesunden HLA-B27⁻-Kontrollpersonen konnte nur eine geringfügige PBL-Proliferation (SI = 2,5) gezeigt werden.

Da bekannt ist, daß einige T-Zellen in Reaktion auf ein Antigen dazu stimuliert werden können, Cytokine zu sezernieren, jedoch nicht proliferieren, wurde auch die Häufigkeit von IFN-γ sezernierenden T-Zellen unter den PBL eines AS-Patienten, der auf das Peptid ansprach, (AS1) und eines auf das Peptid nicht ansprechenden AS-Patienten (AS2) unter Einsatz eines enzymverknüpften Immunospot (ELISPOT)-Assay [modifiziert nach Klinman, D.M., Nutman, T.B., "ELIspot assay to detect cytokine-secreting murine and human cells"; in: Current Protocols in Immunology, 7. Auflage, Hrsg.: Coligan, J.E., Kruisbeek, A.M., Margulies, D.H., Shevach, E.M., Strober, W., Brooklyn, New York, Greene Publishing Associates, 1994] bestimmt. Diese empfindliche ELISPOT-Technik ergab eine spezifische IFN-γ-Produktion durch durch B27PA* stimulierte T-Zellen bei 8 von 10⁵ PBL des Spenders AS1 und bei 1 von 10⁵ PBL des Spenders AS2.

PBL von RA-Patienten wurden von keinem der beiden verwendeten Peptide stimuliert.

Interessanterweise entwickelte der gesunde HLA-B27⁺-Blutspender (bezeichnet als H1), ein 30-jähriger männlicher Blutspender, der innerhalb der Gruppe von gesunden HLA-B27⁺-Probanden die stärkste PBL-Proliferationsantwort auf das Peptid B27PA* (SI = 9,8) zeigte, 8 Monate, nachdem wir die Blutprobe abgenommen hatten, Rückenschmerzen vom entzündlichen Typ und eine morgendliche Steifheit über 1 h. Unglücklicherweise ist dieser Blutspender H1 nicht bereit, sich einer medizinischen Untersuchung durch einen Rheumatologen zu unterziehen.

Wie in nachfolgenden Tabelle gezeigt, ist die B27PA*-spezifische Reaktion der PBL eines AS-Patienten, die auf dieses Peptid ansprachen, dosisabhängig und erreicht ein Maximum bei einer Endkonzentration von 10 µg/ml in Kulturmedium.

| Peptid-Endkonzentration (µg/ml) | B27PA*: cpm ± Standard-abweichung |
|---|---|
| 0 | 165 ± 20 |
| 0,1 | 218 ± 25 |
| 1,0 | 565 ± 47 |
| 5,0 | 1576 ± 70 |
| 7,5 | 2492 ± 72 |
| 10,0 | 2718 ± 76 |
| 20,0 | 2695 ± 88 |
| 50,0 | 2733 ± 84 |

Diese Ergebnisse zeigen, daß ein synthetisches Peptid aus der HLA-B27-Sequenz, B27PA*, als Autoantigen spezifisch durch PBL von HLA-B27⁺-Patienten mit ankylosierender Spondylitis und in einem geringeren Ausmaß durch PBL von HLA-B27⁺-Kontrollpersonen erkannt wird.

In HLA-B27⁻-Kontrollpersonen induzierten die von HLA-B27 abgeleiteten Peptide B27PA* und B27PD keine oder nur geringfügige Proliferation, obwohl diese Peptide für die PBL-Spender fremd waren. Der Mangel an Proliferation gegenüber diesen HLA-B27-Determinanten durch die Kontroll-PBL kann durch die Tatsache erklärt werden, daß diese Art von Reaktivität eine "indirekte Alloerkennung" darstellen würde, die durch Wirts-MHC-restringierte T-Zell-Alloreaktionen gekennzeichnet und bekanntermaßen unveränderlich entweder auf eine einzige oder einige wenige dominante Determinanten auf dem fremden MHC-Antigen gerichtet ist (Benichou, G., Tam, R.C., Loares, L.R.B., Fedoseyeva, E.V., Immunol. Today 18: 67-71, (1997)). Dementsprechend scheinen die hier untersuchten Peptide keine entsprechenden Determinanten auf dem HLA-B27-Molekül zu repräsentieren, die möglicherweise in der Lage wären, alloreaktive T-Zell-Antworten in HLA-B27 - Spendern hervorzurufen.

### Funktionale Charakterisierung von B27PA*-spezifischen T-Zellinien:

Es wurden 3 verschiedene B27PA*-spezifische T-Zellinien durch Inkubieren und Restimulieren von PBL von den Patienten AS1 und AS3 und von einem gesunden HLA-B27⁺-Blutspender (H1) mit dem Peptid B27PA* erzeugt, wie vorstehend beschrieben. AS1 und AS3 waren AS-Patienten, deren PBL in Reaktion auf das Peptid B27PA* stark proliferierten (AS1: SI = 17,5; AS3: SI = 15,4). Die PBL des Blutspenders H1 wurden durch B27PA* mit einem SI von 9,8 stimuliert. Die Kontroll-T-Zellinie B27PA*-AS2 wurden von PBL abgeleitet, die in Reaktion auf B27PA* nicht proliferierten (SI = 1). Die T-Zellinien wurden am Tag 20 der Kultur analysiert. Wie in der nachfolgenden Tabelle gezeigt, proliferierten die Linien B27PA*-AS1, B27PA*-AS3 und B27PA*-H1 spezifisch in Reaktion auf das Peptid B27PA*, wohingegen die Kontrollinie B27PA*-AS2 dies nicht tat. Diese spezifische T-Zell-Proliferation konnte durch anti-MHC-Klasse I-MAbs (w6/32) oder anti-MHC-Klasse II-MAbs (DA6.231) nicht inhibiert werden. Der monoklonaie Antikörper TCR-δ1 (anti-γδ-TCR) war in der Lage, die B27PA*-spezifische Proliferationsreaktion um mehr als 50% in den Kulturen, die mit 1 µg/ml B27PA* stimuliert wurden, zu blockieren, wohingegen er die PHA-induzierte Proliferation nicht beeinflußte.

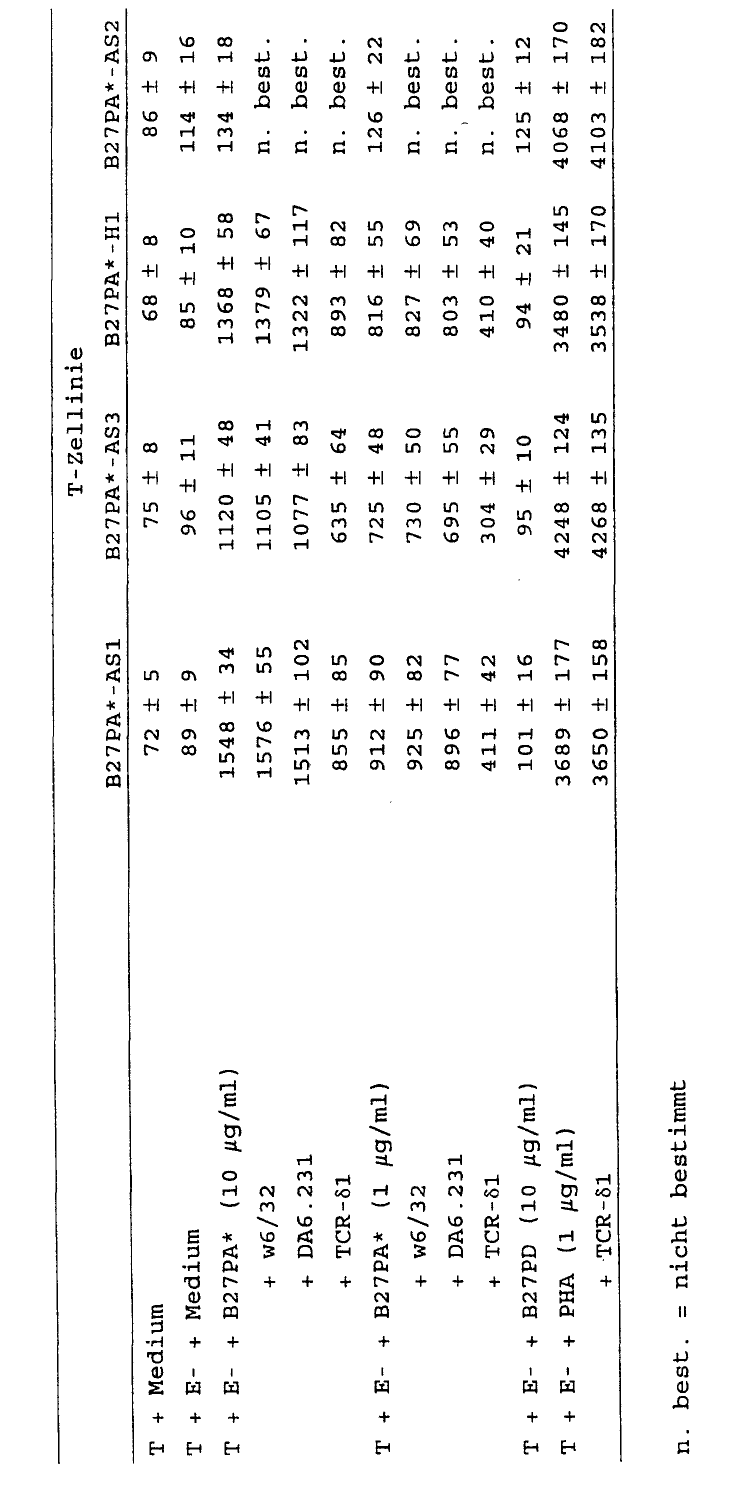

### Durchflußzytometrische Analyse von B27PA*-spezifischen T-Zellinien:

Wie in der nachfolgenden Tabelle zu ersehen ist, ergab eine Phänotypisierung der B27PA*-spezifischen T-Zellinien B27PA*-AS1, B27PA*-AS3 und B27PA*-H1 (ausgeführt an den Tagen 8 und 20 der Kultur) eine bedeutende Expansion von γδ-TCR⁺-Lymphozyten in den B27PA*-stimulierten Linien im Vergleich zu den nicht stimulierten PBL (Tag 0) oder zu nur mit rIL-2 stimulierten PBL-Linien. Am Tag 20 umfaßten γδ-TCR⁺-Lymphozyten 25,5% (Spender AS1), 30,4% (Spender AS3) und 32,7% (Spender H1) der gesamten CD3-positiven Zellpopulation. Im Gegensatz dazu enthielt die durch B27PA* stimulierte Kontrollinie B27PA*-AS2 nur 7,4% γδ-TCR⁺-Zellen. Die in der folgenden Tabelle angegebenen Daten sind als Prozentsatz der gesamten CD3-positiven T-Zellen angegeben.

| | TCR-αβ(%) | CD4 (%) | CD8(%) | TCR-γδ(%) |
|---|---|---|---|---|
| Spender AS1 | | | | |
| frisch isolierte PBL | 93,5 | 69,8 | 24,6 | 5,3 |
| B27PA*-Stimulation, Tag 8 | 84,9 | 52,9 | 28,2 | 13,7 |
| B27PA*-Stimulation, Tag 20 | 73,6 | 45,1 | 30,5 | 25,5 |
| rIL-2-Stimulation, Tag 20 | 93,0 | 67,7 | 26,7 | 6,3 |
| | | | | |

| Spender AS3 | | | | |
|---|---|---|---|---|
| frisch isolierte PBL | 93,9 | 71,0 | 22,4 | 5,8 |
| B27PA*-Stimulation, Tag 8 | 83,6 | 63,5 | 21,3 | 15,5 |
| B27PA*-Stimulation, Tag 20 | 59,2 | 34,4 | 25,6 | 30,4 |
| rIL-2-Stimulation, Tag 20 | 92,2 | 75,8 | 16,6 | 7,2 |
| | | | | |

| Spender H1 | | | | |
|---|---|---|---|---|
| frisch isolierte PBL | 95,7 | 73,1 | 22,3 | 4,1 |
| B27PA*-Stimulation, Tag 8 | 85,2 | 58,4 | 26,5 | 14,0 |
| B27PA*-Stimulation, Tag 20 | 67,1 | 34,9 | 31,5 | 32,7 |
| rIL-2-Stimulation, Tag 20 | 93,7 | 75,0 | 18,4 | 6,0 |
| | | | | |

| Spender AS2 | | | | |
|---|---|---|---|---|
| frisch isolierte PBL | 94,1 | 66,3 | 27,4 | 5,3 |
| B27PA*-Stimulation, Tag 8 | 92,3 | 68,6 | 24,0 | 7,2 |
| B27PA*-Stimulation, Tag 20 | 91,9 | 70,2 | 21,4 | 7,8 |
| rIL-2-Stimulation, Tag 20 | 92,1 | 74,3 | 18,5 | 7,4 |

Die aufgeführten Daten aus der FACScan-Analyse und aus MAb-Blockierungsexperimenten von B27PA*-spezifischen T-Zellinien zeigen, daß nicht CD4-positive αβ-TCR⁺-T-Zellen, sondern vielmehr γδ-TCR⁺-T-Zellen die Hauptpopulation waren, die nach einer in vitro-Inkubation mit dem von HLA-B27 abgeleiteten Peptid stimuliert und expandiert wurde. In diesem Zusammenhang ist es erwähnenswert, daß bei mehreren humanen Autoimmunerkrankungen, einschließlich rheumatoider Arthritis vermutet wurde (Söderström, J., Halapi, E., Nilsson, E., Grönberg, A., van Embden, J., Klareskog, L., Kiessling, R. Scand. J. Immunol. 32, 503-515, (1990)), daß selbstreaktive γδ-TCR⁺-Zellen an der Pathogenese der Erkrankung beteiligt sind, obwohl die relative Anzahl von γδ-TCR⁺-Zellen weder im peripheren Blut noch in den Synovialflüssigkeiten und Membranen von RA-Patienten erhöht sind (Smith, M., Bröker, B., Moretta, L., Ciccone, E., Grossi, C.E., Edwards, J.C.W., Yüksel, F., Colaco, B., Worman, C., Mackenzie, L., Kinne, R., Weseloh, G., Glückert, K., Lydyard, P.M., Scand. J. Immunol. 32, 585-593 (1990)). Bei den Spondylarthropathien wurden γδ-TCR⁺-Zellen aus Synovialflüssigkeit isoliert, die in Reaktion auf *Klebsiella pneumoniae* proliferierten (Hermann, E., Sucké, B., Droste, U., Meyer zum Büschenfelde, K.-H., Arthritis Rheum. 38, 1277-1282, (1995)) oder Cytotoxizität gegen Selbst-Ziele, Daudi-Zellinien oder gegen durch Enterobakterien infizierte B-Zellinien zeigten (Hermann, E., Ackermann, B., Duchmann, R., Meyer zum Büschenfelde, K.-H., Clin. Exp. Rheumatol. 13, 187-191, (1995)). Hinsichtlich der Pathogenese von Spondylarthropathien wurden dementsprechend Spekulationen angestellt, daß γδ-TCR⁺-Zellen eine Rolle bei den frühen, gegen durch arthritogene Bakterien infizierte Zellen oder gegen autologe entzündete "gestreßte" Gewebe gerichteten Abwehrmechanismen spielen könnten (Hermann, E., Ackermann, B., Duchmann, R., Meyer zum Büschenfelde, K.-H., Clin. Exp. Rheumatol. 13, 187-191, (1995)). Eine andere wichtige Beobachtung wurde hinsichtlich der Rolle von HLA-B27-Peptid-spezifischen γδ-TCR⁺-Zellen im Tiermodell der experimentellen Autoimmun-Uveitis (EAU) gemacht. Adoptiv transferierte γδ-TCR⁺-Zellen von Ratten, bei denen mit dem uveitogenen autoantigenen S-Ag-Peptid oder dem kreuzreaktiven Peptid B27PD oral eine Toleranz induziert worden war, konnten eine Suppression der Uveitis in antigen-spezifischer Weise vermitteln (Wildner, G., Hünig, T., Thurau, S., Eur. J. Immunol. 26, 2140-48, (1996)). Angesichts dieser Ergebnisse ist es denkbar, daß auch bei der menschlichen Krankheit der ankylosierenden Spondylitis γδ-TCR⁺-Zellen mit einer Spezifität für ein Peptid aus dem mit der Krankheit assoziierten HLA-B-Antigen B27 an der Immunmodulation oder sogar der Herunterregulierung von Entzündungen beteiligt sein könnten. Die bei der vorstehend beschriebenen Untersuchung gemachte Beobachtung einer Reaktivität peripherer T-Zellen gegenüber dem Peptid B27PA* bei gesunden, HLA-B27-positiven Blutspendern steht der letztgenannten Möglichkeit nicht entgegen.

## Patentansprüche

1. Verwendung eines oder mehrerer Peptide mit einer der folgenden allgemeinen Sequenzen zur Bereitstellung/Herstellung eines Mittels zur Diagnose und/oder Behandlung von HLA-abhängigen Autoimmunkrankheiten:
BL₁-X-BL₂-Y-BL₃-Z
oder
BL₁-X-BL₂-Y-BL₃-Z-BL₄-W-BL₅
wobei BL₁ für 1 bis 2 Aminosäuren steht,
X für Asp steht,
BL₂ für 4 bis 5 Aminosäuren steht,
Y für Ile steht,
BL₃ für 3 bis 4 Aminosäuren steht,
Z für Lys-Ala-Gln oder Lys-Ala-Glu steht,
BL₄ für 6 bis 8 Aminosäuren steht,
W für Arg steht,
Bl₅ für 1 bis 2 Aminosäuren steht,
wobei ggf. C und/oder N-terminal weitere Aminosäurereste, Glykosylreste, Fettsäurereste oder Alkyl/Alkenylreste (C₁-C₁₆) vorhanden sind.

2. Verwendung nach Anspruch 1, wobei das folgende Peptid 
Trp-Asp-Arg-Glu-Thr-Gln-Ile-Cys-Lys-Ala-Lys-Ala-Gln-Thr-
Asp-Arg-Glu-Asn-Leu-Arg-Thr
 oder ein durch Substitution, Modifikation, Insertion und/oder terminale Anfügung einzelner Aminosäuren oder kleinerer Gruppen von Aminosäuren von der vorstehend angegebenen Sequenz abgeleitetes Peptid (es gelten die Definitionen gemäß Anspruch 1) verwendet wird.

3. Verwendung nach Anspruch 1, wobei das folgende Peptid Trp-Asp-Arg-Glu-Thr-Gln-Ile-Cys-Lys-Ala-Lys-Ala-Gln oder ein durch Substitution, Modifikation, Insertion und/oder terminale Anfügung einzelner Aminosäuren oder kleinerer Gruppen von Aminosäuren von der vorstehend angegebenen Sequenz abgeleitetes Peptid (es gelten die Definitionen gemäß Anspruch 1) verwendet wird.

4. Verwendung nach Anspruch 1, wobei das folgende Peptid 
Leu-Asp-Leu-Asp-Ser-Ile-Ile-Ala-Glu-Val-Lys-Ala-Gln-Tyr-
Glu-Glu-Ile-Ala-Asn-Arg-Ser
 oder ein durch Substitution, Modifikation, Insertion und/oder terminale Anfügung einzelner Aminosäuren oder kleinerer Gruppen von Aminosäuren von der vorstehend angegebenen Sequenz abgeleitetes Peptid (es gelten die Definitionen gemäß Anspruch 1) verwendet wird.

5. Verwendung eines oder mehrerer Peptide mit der folgenden Sequenz zur Bereitstellung/Herstellung eines Mittels zur Diagnose und/oder Behandlung von HLA-abhängigen Autoimmunkrankheiten:
BL₆-O-BL₇-M-BL₈
wobei BL₆ für 1 Aminosäure steht,
O für Leu-Arg steht,
BL₇ für 1 Aminosäure steht,
M für Tyr steht und
BL₈ für 0 bis 5 Aminosäuren steht,
wobei ggf. C und/oder N-terminal Glykosylreste, Fettsäurereste oder Alkyl/Alkenylreste (C₁-C₁₆), oder terminale Aufügung einzelner Aminosäuren oder kleinerer Gruppen von Aminosäuren vorhanden sind.

6. Verwendung nach Anspruch 5, wobei das folgende Peptid Leu-Leu-Arg-Gly-Tyr-His-Gln-Asp-Ala-Tyr oder ein durch Substitution, Modifikation, Insertion und/oder terminale Anfügung einzelner Aminosäuren oder kleinerer Gruppen von Aminosäuren von der vorstehend angegebenen Sequenz abgeleitetes Peptid (es gelten die Definitionen gemäß Anspruch 5) verwendet wird.

7. Verwendung nach Anspruch 5, wobei das folgende Peptid 
Arg-Val-Ala-Glu-Gln-Leu-Arg-Ala-Tyr-Leu-Glu-Gly-Glu-Cys-
Val
 oder ein durch Substitution, Modifikation, Insertion und/oder terminale Anfügung einzelner Aminosäuren oder kleinerer Gruppen von Aminosäuren von der vorstehend angegebenen Sequenz abgeleitetes Peptid (es gelten die Definitionen gemäß Anspruch 5) verwendet wird.

8. Verwendung nach einer der vorstehenden Ansprüche, wobei die HLA-abhängige Autoimmunerkrankung ankylosierender Spondylitis (Morbus Bechterew), Arthritis psoriatica, juveniler rheumatoider Arthritis, Iritis, Uveitis oder Psoriasis ist.

9. Kit zur Verwendung in einem Assay zur Diagnose von HLA-abhängigen Autoimmunerkrankungen, der neben anderen, für den Assay benötigten Reagenzien eines oder mehrere der Peptide, wie sie in den Ansprüchen 1 bis 7 definiert sind, umfaßt.

10. Peptid mit der folgenden Sequenz: 
Leu-Asp-Leu-Asp-Ser-Ile-Ile-Ala-Glu-Val-Lys-Ala-Gln-Tyr-
Glu-Glu-Ile-Ala-Asn-Arg-Ser
 oder einer durch Substitution, Modifikation, Insertion und/oder terminale Anfügung einzelner Aminosäuren oder kleinerer Gruppen von Aminosäuren von der vorstehend angegebenen Sequenz abgeleiteten Sequenz (es gelten die Definitionen gemäß Anspruch 1).

11. Peptid mit der folgenden Sequenz: 
Arg-Val-Ala-Glu-Gln-Leu-Arg-Ala-Tyr-Leu-Glu-Gly-Glu-Cys-
Val
 oder einer durch Substitution, Modifikation, Insertion und/oder terminale Anfügung einzelner Aminosäuren oder kleinerer Gruppen von Aminosäuren von der vorstehend angegebenen Sequenz abgeleiteten Sequenz (es gelten die Definitionen gemäß Anspruch 5).

## Claims

1. Use of one or more peptides having one of the following general sequences for the provision/manufacture of a medicament for the diagnosis and/or treatment of HLA-dependent autoimmune diseases:
BL₁-X-BL₂-Y-BL₃-Z
or
BL₁-X-BL₂-Y-BL₃-Z-BL₄-W-BL₅
where BL₁ represents from 1 to 2 amino acids,
X represents Asp,
BL₂ represents from 4 to 5 amino acids,
Y represents Ile,
BL₃ represents from 3 to 4 amino acids,
Z represents Lys-Ala-Gln or Lys-Ala-Glu,
BL₄ represents from 6 to 8 amino acids,
W represents Arg,
BL₅ represents from 1 to 2 amino acids,
wherein, where appropriate, further amino acid residues, glycosyl residues, fatty acid residues or alkyl/alkenyl residues (C₁-C₁₆) are present C-terminally and/or N-terminally.

2. Use according to Claim 1, where the following peptide 
Trp-Asp-Arg-Glu-Thr-Gln-Ile-Cys-Lys-Ala-Lys-Ala-Gln-Thr-Asp-Arg-Glu-Asn-Leu-Arg-
Thr
 or a peptide which is derived from the abovementioned sequence (the definitions according to Claim 1 apply) by substitution, modification, insertion and/or terminal addition of individual amino acids or relatively small groups of amino acids, is used.

3. Use according to Claim 1, where the following peptide Trp-Asp-Arg-Glu-Thr-Gln-Ile-Cys-Lys-Ala-Lys-Ala-Gln or a peptide which is derived from the abovementioned sequence (the definitions according to Claim 1 apply) by substitution, modification, deletion, insertion and/or terminal addition of individual amino acids or relatively small groups of amino acids, is used.

4. Use according to Claim 1, where the following peptide Leu-Asp-Leu-Asp-Ser-Ile-Ile-Ala-Glu-Val-Lys-Ala-Gln-Tyr-Glu-Glu-Ile-Ala-Asn-Arg-Ser or a peptide which is derived from the abovementioned sequence (the definitions according to Claim 1 apply) by substitution, modification, insertion and/or terminal addition of individual amino acids or relatively small groups of amino acids, is used.

5. Use of one or more peptides having the following sequence for the provision/manufacture of a medicament for the diagnosis and/or treatment of HLA-dependent autoimmune diseases:
BL₆-O-BL₇-M-BL₈
where BL₆ represents 1 amino acid,
O represents Leu-Arg,
BL₇ represents 1 amino acid,
M represents Tyr, and
BL₈ represents from 0 to 5 amino acids,
wherein, where appropriate, glycosyl residues, fatty acid residues or alkyl/alkenyl residues (C₁-C₁₆) are present C-terminally and/or N-terminally, or terminal addition of individual amino acids.

6. Use according to Claim 5, where the following peptide Leu-Leu-Arg-Gly-Tyr-His-Gln-Asp-Ala-Tyr or a peptide which is derived from the abovementioned sequence (the definitions according to Claim 5 apply) by substitution, modification, insertion and/or terminal addition of individual amino acids or relatively small groups of amino acids, is used.

7. Use of a peptide according to claim 5, where the following peptide: Arg-Val-Ala-Glu-Gln-Leu-Arg-Ala-Tyr-Leu-Glu-Gly-Glu-Cys-Val or a peptide which is derived from the abovementioned sequence (the definitions according to Claim 5 apply) by substitution, modification, insertion and/or terminal addition of individual amino acids or relatively small groups of amino acids, is used.

8. Use according to one of the preceding claims, wherein the HLA-dependent antoimmune disease is ankylosing spondylitis (Bechterew's disease), psoriatic arthritis, juvenile rheumatoid arthritis, iritis, uveitis or psoriasis.

9. Kit for use in an assay for diagnosing HLA-dependent autoimmune diseases, which comprises one or more of the peptides as defined in Claims 1 to 7 in addition to other reagents which are required for the assay.

10. Peptide having the following sequence: Leu-Asp-Leu-Asp-Ser-Ile-Ile-Ala-Glu-Val-Lys-Ala-Gln-Tyr-Glu-Glu-Ile-Ala-Asn-Arg-Ser or a sequence which is derived from the abovementioned sequence (the definitions according to Claim 1 apply) by substitution, modification, insertion and/or terminal addition of individual amino acids or relatively small groups of amino acids.

11. Peptide having the following sequence: Arg-Val-Ala-Glu-Gln-Leu-Arg-Ala-Tyr-Leu-Glu-Gly-Glu-Cys-Val or a sequence which is derived from the abovementioned sequence (the definitions according to Claim 5 apply) by substitution, modification, insertion and/or terminal addition of individual amino acids or relatively small groups of amino acids.

## Revendications

1. Utilisation d'un ou plusieurs peptides ayant l'une des séquences générales suivantes pour la préparation/production d'un agent pour le diagnostic et/ou le traitement de maladies auto-immunes dépendantes de HLA :
BL₁-X-BL₂-Y-BL₃-Z
ou
BL₁-X-BL₂-Y-BL₃-Z-BL₄-W-BL₅
où BL₁ représente 1 à 2 acides aminés,
X représente Asp,
BL₂ représente 4 à 5 acides aminés,
Y représente Ile,
BL₃ représente 3 à 4 acides aminés,
Z représente Lys-Ala-Gln ou Lys-Ala-Glu,
BL₄ représente 6 à 8 acides aminés,
W représente Arg,
BL₅ représente 1 à 2 acides aminés,
où éventuellement d'autres restes d'acides aminés, des restes glycosyle, des restes d'acides gras ou des restes alkyle/alcényle (C₁-C₁₆) sont présents en position C- et/ou N-terminale.

2. Utilisation selon la revendication 1, où le peptide suivant 
Trp-Asp-Arg-Glu-Thr-Gln-Ile-Cys-Lys-Ala-Lys-Ala-Gln-Thr-Asp-Arg-Glu-Asn-
Leu-Arg-Thr
 ou un peptide dérivé de la séquence indiquée précédemment par substitution, modification, insertion et/ou adjonction terminale d'acides aminés individuels ou de groupes relativement petits d'acides aminés (les définitions selon la revendication 1 sont valables) est utilisé.

3. Utilisation selon la revendication 1, où le peptide suivant Trp-Asp-Arg-Glu-Thr-Gln-Ile-Cys-Lys-Ala-Lys-Ala-Gln ou un peptide dérivé de la séquence indiquée précédemment par substitution, modification, insertion et/ou adjonction terminale d'acides aminés individuels ou de groupes relativement petits d'acides aminés (les définitions selon la revendication 1 sont valables) est utilisé.

4. Utilisation selon la revendication 1, où le peptide suivant 
Leu-Asp-Leu-Asp-Ser-Ile-Ile-Ala-Glu-Val-Lys-Ala-Gln-Tyr-Glu-Glu-Ile-Ala-
Asn-Arg-Ser
 ou un peptide dérivé de la séquence indiquée précédemment par substitution, modification, insertion et/ou adjonction terminale d'acides aminés individuels ou de groupes relativement petits d'acides aminés (les définitions selon la revendication 1 sont valables) est utilisé.

5. Utilisation d'un ou plusieurs peptides ayant la séquence suivante pour la préparation/production d'un agent pour le diagnostic et/ou le traitement de maladies auto-immunes dépendantes de HLA :
BL₆-O-BL₇-M-BL₈
où BL₆ représente un acide aminé,
O représente Leu-Arg,
BL₇ représente un acide aminé,
M représente Tyr et
BL₈ représente 0 à 5 acides aminés,
où éventuellement des restes glycosyle, des restes d'acides gras ou des restes alkyle/alcényle (C₁-C₁₆) ou l'adjonction terminale d'acides aminés individuels ou de groupes relativement petits d'acides aminés sont présents en position C- et/ou N-terminale.

6. Utilisation selon la revendication 5, où le peptide suivant Leu-Leu-Arg-Gly-Tyr-His-Gln-Asp-Ala-Tyr ou un peptide dérivé de la séquence indiquée précédemment par substitution, modification, insertion et/ou adjonction terminale d'acides aminés individuels ou de groupes relativement petits d'acides aminés (les définitions selon la revendication 5 sont valables) est utilisé.

7. Utilisation selon la revendication 5, où le peptide suivant Arg-Val-Ala-Glu-Gln-Leu-Arg-Ala-Tyr-Leu-Glu-Gly-Glu-Cys-Val ou un peptide dérivé de la séquence indiquée précédemment par substitution, modification, insertion et/ou adjonction terminale d'acides aminés individuels ou de groupes relativement petits d'acides aminés (les définitions selon la revendication 5 sont valables) est utilisé.

8. Utilisation selon l'une des revendications précédentes, où la maladie auto-immune dépendante de HLA est la spondylarthrite ankylosante (maladie de Bechterew), le rhumatisme psoriasique, la polyarthrite rhumatoïde juvénile, l'iritis, l'uvéite ou le psoriasis.

9. Trousse destinée à être utilisée dans un test pour le diagnostic de maladies auto-immunes dépendantes de HLA qui comprend, outre d'autres réactifs nécessaires pour le test, un ou plusieurs des peptides définis dans les revendications 1 à 7.

10. Peptide ayant la séquence suivante : 
Leu-Asp-Leu-Asp-Ser-Ile-Ile-Ala-Glu-Val-Lys-Ala-Gln-Tyr-Glu-Glu-Ile-Ala-
Asn-Arg-Ser
 ou une séquence dérivée de la séquence indiquée précédemment par substitution, modification, insertion et/ou adjonction terminale d'acides aminés individuels ou de groupes relativement petits d'acides aminés (les définitions selon la revendication 1 sont valables).

11. Peptide ayant la séquence suivante : Arg-Val-Ala-Glu-Gln-Leu-Arg-Ala-Tyr-Leu-Glu-Gly-Glu-Cys-Val ou une séquence dérivée de la séquence indiquée précédemment par substitution, modification, insertion et/ou adjonction terminale d'acides aminés individuels ou de groupes relativement petits d'acides aminés (les définitions selon la revendication 5 sont valables).
